(19) **Europäisches Patentamt / European Patent Office / Office européen des brevets**

(11) **EP 1 528 891 B1**

(12) **EUROPEAN PATENT SPECIFICATION**

(45) Date of publication and mention
of the grant of the patent:
**31.10.2007   Bulletin 2007/44**

(51) Int Cl.:
*A61B 5/15* (2006.01)   *A61B 5/00* (2006.01)

(21) Application number: **04776356.0**

(22) Date of filing: **07.06.2004**

(86) International application number:
**PCT/US2004/018144**

(87) International publication number:
**WO 2004/107977 (16.12.2004 Gazette 2004/51)**

(54) **SYSTEMS FOR EXTRACTING BODILY FLUID AND MONITORING AN ANALYTE THEREIN**

VORRICHTUNG ZUR ENTNAHME VON KÖRPERFLÜSSIGKEITEN UND ZUR ANALYTENÜBERWACHUNG

SYSTEMES D'EXTRACTION D'UN FLUIDE CORPOREL ET DE SURVEILLANCE D'UN ANALYTE PRESENT DANS LEDIT FLUIDE

(84) Designated Contracting States:
**AT BE BG CH CY CZ DE DK EE ES FI FR GB GR HU IE IT LI LU MC NL PL PT RO SE SI SK TR**

(30) Priority: **06.06.2003   US 476733 P
28.08.2003   US 653023
04.06.2004   US 861749**

(43) Date of publication of application:
**11.05.2005   Bulletin 2005/19**

(73) Proprietor: **LifeScan, Inc.
Milpitas, CA 95035 (US)**

(72) Inventors:
• **RACCHINI, Joel
Edina, MN 55424 (US)**
• **STOUT, Phil
Roseville, MN 55113 (US)**
• **HILGERS, Michael, Edward
Lake Elmo, MN 55042 (US)**
• **RADEMACHER, Thomas
Blaine, MN 55449 (US)**
• **MECHELKE, Joel
Stillwater, MN 55082 (US)**
• **HANSON, Cass, A.
St. Paul, MN 55104 (US)**

(74) Representative: **Mercer, Christopher Paul
Carpmaels & Ransford
43-45 Bloomsbury Square
London WC1A 2RA (GB)**

(56) References cited:
EP-A- 1 260 815          US-A1- 2002 022 789
US-A1- 2002 082 522          US-A1- 2003 073 931

**Description**

**BACKGROUND OF INVENTION**

1. Field of the Invention

The present invention relates, in general, to medical devices and, in particular, to devices, and systems for extracting bodily fluid and monitoring an analyte therein.

2. Description of the Related Art

In recent years, efforts in medical devices for monitoring analytes (e.g., glucose) in bodily fluids (e.g., blood and interstitial fluid) have been directed toward developing devices and methods with reduced user discomfort and/or pain, simplifying monitoring methods and developing devices and methods that allow continuous or semi-continuous monitoring. Simplification of monitoring methods enables users to self-monitor such analytes at home or in other locations without the help of health care professionals. A reduction in a user's discomfort and/or pain is particularly important in devices and methods designed for home use in order to encourage frequent and regular use. It is thought that if a blood glucose monitoring device and associated method are relatively painless, users will monitor their blood glucose levels more frequently and regularly than otherwise.

In the context of blood glucose monitoring, continuous or semi-continuous monitoring devices and methods are advantageous in that they provide enhanced insight into blood glucose concentration trends, the effect of food and medication on blood glucose concentration and a user's overall glycemic control. In practice, however, continuous and semi-continuous monitoring devices can have drawbacks. For example, during extraction of an interstitial fluid (ISF) sample from a target site (e.g., a target site in a user's skin layer), ISF flow rate can decay over time. Furthermore, after several hours of continuous ISF extraction, a user's pain and/or discomfort can increase significantly and persistent blemishes can be created at the target site.

EP-A-1260 815 teaches a method and an associated system for extracting an ISF sample and monitoring an analyte therein. The system comprises a disposable cartridge containing a sampling module and an analysis module, and a local controller module in electronic communication with the cartridge.

US 2002/0022789 teaches an ISF extraction device including a pressure ring for applying pressure to stimulate the area in the vicinity of a target site.

Still needed in the field, therefore, is a device and associated method for the monitoring of an analyte (e.g., glucose) in a bodily fluid (such as ISF) that is simple to employ, creates relatively little discomfort and/or pain in a user, and facilitates continuous or semi-continuous monitoring without unduly increasing a user's pain or creating persistent blemishes.

US 2002/0082522 discloses a system of the type set out in the preamble of the accompanying claim 1.

**SUMMARY OF INVENTION**

Systems for the extraction of a bodily fluid sample and monitoring of an analyte therein according to embodiments of the present invention are simple to employ, create relatively little pain and/or discomfort in a user, and facilitate continuous and semi-continuous monitoring without unduly increasing a user's pain or creating persistent blemishes. In addition, ISF extraction devices according to embodiments of the present invention also create relatively little pain and/or discomfort in a user and facilitate continuous and semi-continuous monitoring without unduly increasing a user's pain or creating persistent blemishes.

A system for extracting an Interstitial fluid (ISF) sample and monitoring an analyte therein according to an exemplary embodiment of the present invention includes a cartridge (e.g., a disposable cartridge) and a local controller module. The cartridge includes a sampling module adapted to extract an ISF sample from a target site of a body and an analysis module adapted to measure an analyte (e.g., glucose) in the ISF sample. In addition, the local controller module is in electronic communication with the cartridge and is configured to receive and store measurement data (e.g., a current signal) from the analysis module.

The sampling module also includes at least one pressure ring adapted for applying pressure to the body in the vicinity of the target site. In addition, the sampling module is configured such that the at least one pressure ring is capable of applying the pressure in an oscillating manner whereby an ISF glucose lag of the ISF sample extracted by the penetration member is mitigated.

The sampling module further includes a penetration member which is fixed with respect to the at least one pressure ring.

The sampling module of systems according to embodiments of the present invention can optionally includes a penetration member configured for penetrating a target site of a user's skin layer and, subsequently, residing in the user's skin layer and extracting an ISF sample therefrom. Alternatively, the sampling module can employ microdialysis, ultrafiltration, laser, reverse iontophoresis, electroporation and/or ultrasound techniques to extract a sample (e.g., an ISF sample) from a target site of a user.

In addition to, or as an alternative to, a pressure ring(s) that is capable of applying pressure in an oscillating manner, other ISF glucose lag mitigating techniques can be employed in embodiments of the present invention. Such ISF glucose lag mitigating techniques include the use of lag mitigating chemicals, the use of heat, ultrasound, non-oscillating mechanical manipulation, vacuum, electropotential and combinations thereof to mitigate ISF glucose lag.

The cartridge may be disposable. The disposable nature of a disposable cartridge renders systems according to embodiments of the present invention simple to employ. In addition, when a pressure ring is operated in an oscillating manner according to the present invention, continuous and semi-continuous monitoring is facilitated while simultaneously minimizing a user's pain and the creation of persistent blemishes.

Since the penetration member of ISF extraction devices according to the present invention can reside in a user's skin layer during extraction of an ISF sample, the ISF extraction devices are simple to employ. In addition, since the ISF extraction device is configured to apply pressure in an oscillating manner, continuous and semi-continuous monitoring is facilitated while minimizing a user's pain and the creation of persistent blemishes. Application of pressure in an oscillating manner by the pressure ring(s) can also optimize blood flow to the vicinity of the target site such that ISF glucose lag is minimized.

## BRIEF DESCRIPTION OF DRAWINGS

A better understanding of the features and advantages of the present invention will be obtained by reference to the following detailed description that sets forth illustrative embodiments, in which principles of the invention are utilized, and the accompanying drawings of which:

FIG. 1 is a simplified block diagram depicting a system for extracting a bodily fluid sample and monitoring an analyte therein according to an exemplary embodiment of the present invention;

FIG. 2 is a simplified schematic diagram of an ISF sampling module according to an exemplary embodiment of the present invention being applied to a user's skin layer, with the dashed arrow indicating a mechanical interaction and the solid arrows indicating ISF flow or, when associated with element 28, the application of pressure;

FIG. 3 is a simplified block diagram of an analysis module and local controller module according to an exemplary embodiment the present invention;

FIG. 4 is a simplified block diagram of an analysis module, local controller module and remote controller module according to an exemplary embodiment of the present invention;

FIG. 5 is a simplified block diagram of a remote controller module according to an exemplary embodiment of the present invention;

FIG. 6 is a top perspective view of a disposable cartridge and local controller module according to an exemplary embodiment of the present invention;

FIG. 7 is a bottom perspective view of the disposable cartridge and local controller module of FIG. 6;

FIG. 8 is a perspective view of a system according to another exemplary embodiment of the present invention with the disposable cartridge and local controller module attached to an arm of a user;

FIG. 9 is a simplified cross-sectional side view of an extraction device according to an exemplary embodiment of the present invention;

FIG. 10 is a perspective view of a portion of an extraction device according to yet another exemplary embodiment of the present invention;

FIG. 11 is a simplified cross-sectional side view of the extraction device of FIG. 10;

FIG. 12 is a graph showing perfusion as a function of time for a test conducted using the extraction device of FIG. 9;

FIG. 13 is a flow diagram illustrating a sequence of steps in a process according to one exemplary embodiment of the present invention;

FIG. 14 is a simplified cross-sectional side view of a portion of an extraction device according a further embodiment of the present invention:

FIG 15 is a time course plot of glucose concentration versus time depicting glucose profiles determined from finger capillary blood, control ISF samples and test ISF samples;

FIG. 16A and 16B depict regressions superimposed on Clarke Error Grids for control ISF glucose versus finger capillary blood glucose and test ISF glucose versus finger capillary blood glucose, respectively;

FIG. 17 is a plot of percentage bias versus relative time for both test ISF and control ISF glucose measurements;

FIG. 18 is a regression superimposed on a Clarke Error Grid for bias corrected test ISF glucose versus finger capillary blood glucose; and

FIGs. 19A and 19B are error, as %RMS(CV) versus time lag for control ISF and test ISF, respectively.

## DETAILED DESCRIPTION OF THE INVENTION

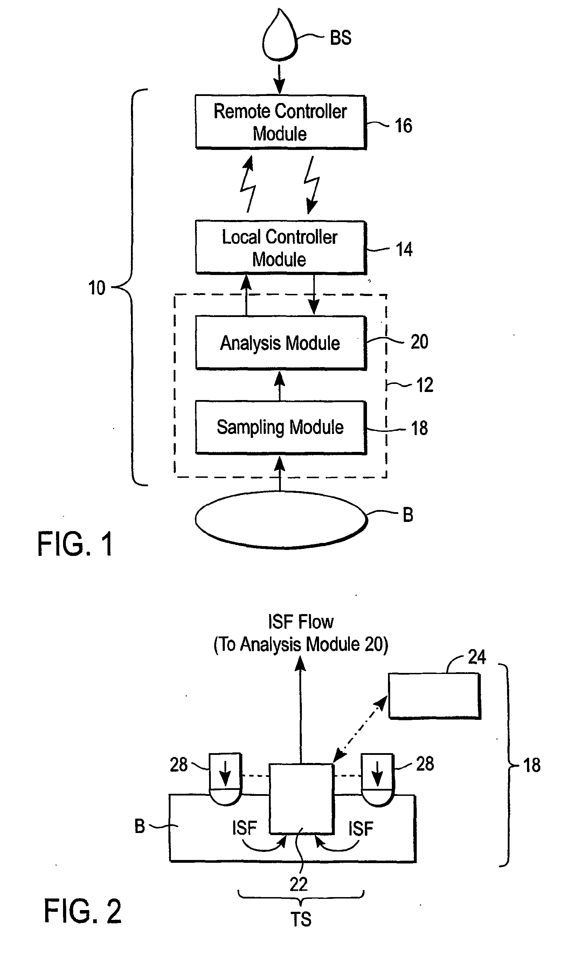

A system 10 for extracting a bodily fluid sample (e.g., an ISF sample) and monitoring an analyte (for example, glucose) therein according to an exemplary embodiment of the present invention includes a disposable cartridge 12 (encompassed within the dashed box), a local controller module 14, and a remote controller module 16, as illustrated in FIG. 1.

In system 10, disposable cartridge 12 includes a sampling module 18 for extracting the bodily fluid sample (namely, an ISF sample) from a body (B, for example a user's skin layer) and an analysis module 20 for measuring an analyte (i.e., glucose) in the bodily fluid. Sampling module 18 and analysis module 20 can be any suitable sampling and analysis modules known to those of skill in the art. It should be noted that sampling module 18 and analysis module 20 of system 10 are both configured to be disposable since they are components of disposable cartridge 12. However, it should also be noted that embodiments of systems according to the present invention can alternatively employ a cartridge that is not disposable (i.e., simply a "cartridge" as opposed to a "disposable cartridge").

Sampling module 18 could employ any suitable technique to extract the bodily fluid sample including, but not limited to, the microdialysis, ultrafiltration, laser, reverse iontophoresis, electroporation, and ultrasound techniques described below and combinations thereof, although these fall outside the scope of the claimed invention. According to the invention, the sampling module 18 employs a penetration member (e.g., a needle) to extract the bodily fluid.

Two techniques for extracting a bodily fluid sample (e.g., ISF) that could be used by sampling modules, but are outside the scope of the present invention, are microdialysis and ultrafiltration. Microdialysis and ultrafiltration techniques can, for example, employ a tubular-shaped semi-permeable membrane having a first end, a second end and pores that allow low molecular weight chemical compounds (e.g., glucose) to diffuse through, or otherwise migrate across, the semi-permeable membrane. However, the pore size and/or geometry is predetermined to prevent high molecular weight chemical compounds (such as proteins) from diffusing through or migrating across the semi-permeable membrane.

Suitable semi-permeable membrane materials include, but are not limited to, polyacrylonitrile, cuprophan, regenerated cellulose, polycarbonate and polysulfone. During use, the tubular semi-permeable membrane is, for example, implanted into the subcutaneous skin layer of a user's body.

In microdialysis, a perfusion solution is pumped into the first end such that the perfusion solution flows through the inside of the tubing, where various small molecular weight chemical compounds (such as glucose) that have diffused through or migrated across the semi-permeable membrane enter the perfusion solution. The perfusion solution flows to the second end. The perfusion solution and the various small molecular weight chemical compounds can then be transferred to, and analyzed by, analysis module 20.

In ultrafiltration, a relatively low (i.e., "negative") pressure is applied to both the first end and second end, causing bodily fluid (e.g., ISF) to migrate by filtration across the semi-permeable membrane and flow towards the first and second end of the tubing. The resulting ultrafiltrate (e.g., ISF ultrafiltrate) can then be transferred to, and analyzed by, analysis module 20.

If desired, the tubular-shaped semi-permeable membrane can be fused to a catheter or cannula to facilitate insertion and handling. Further details regarding microdialysis and ultrafiltration are in U.S Patents No.'s 5,002,054, 5,706,806 and 5,174,291.

Another technique for extracting ISF which may be employed by sampling module 18, but which is outside the scope of the present invention, is a laser. The use of a laser provides many advantages, including the ability to create a small puncture or localized erosion of the skin tissue, without a large degree of concomitant pain. For example, a narrowly focused laser may be adapted to ablate a user's skin layer such that a micropore is formed therein and ISF is caused to be expressed. Because the depth of ablation can be tightly controlled with a laser, the process of extracting ISF can in theory be painless and such that the ISF is sufficiently free of blood. The power level, wavelength range, optics, and pulse frequency of the laser may be adapted so as to increase the efficiency of ablation. More details in regards to the use of a laser in collecting ISF can be found in U.S. Patent No. 5,165,418 and International Publication No. WO 97/07734.

By using reverse iontophoresis technique, an iontophoresed ISF sample may be extracted by employing sampling module 18. This technique, which is outside the scope of the present invention, relies on movement of ISF and glucose across a user's skin layer by way of applied electric potential or current. Iontophoresis involves, for example, a pair of iontophoretic electrodes (which are coated with a hydrogel) being mounted onto the user's skin layer in a spaced apart arrangement. A current density of, for example, about 0.01 to about 0.5 $mA/cm^2$ is then applied between the two electrodes. Typically, the polarity of the applied current will be switched about every 10 minutes to increase the flux of the iontophoresed ISF sample across the user's skin layer. The application of current causes the iontophoresed ISF sample to be expressed from the user's skin layer because of electro-osmotic forces. Adjacent to the iontophoretic electrodes, a reservoir is provided to collect the iontophoresed ISF samples so that they can be subsequently analyzed by analysis module 20. More details in regards to the use of reverse iontophoresis can be found in U.S Patent No.'s 6,233,471 and 6,272,364.

Yet another technique for extracting ISF which may be employed with sampling module 18 is electroporation. This technique is also outside the scope of the present invention. Electroporation initially involves forming at least one micropore to a predetermined depth through a user's skin layer. The method for forming the at least one micropore may use a laser or heated wire. Next, an electrical voltage is applied between an electrode electrically coupled to the micropore and another electrode spaced therefrom.

By applying electrical voltage to the user's skin layer that has been breached by a micropore, electroporation effects can be targeted at tissue structures beneath the surface, such as capillaries, to greatly enhance the withdrawal of biological fluid. A means for collecting and transferring ISF can be provided so that ISF samples extracted by electroporation can then be subsequently analyzed by analysis module 20. More details in regards to electroporation can be found in U.S Patent No. 6,022,316.

Still another technique for extracting ISF which may be used with sampling module 18 is ultrasound. This technique is also outside the scope of the present invention. This technique focuses an ultrasound beam onto a small area of a user's skin layer. The number of pain receptors within the ultrasound application site decreases as the application area decreases. Thus, the application of ultrasound to a very small area will produce less sensation and will allow ultrasound and/or its local effects to be administered at higher intensities with little pain or discomfort. Large forces can be produced locally, resulting in cavitations, mechanical oscillations in the skin itself, and large localized shearing forces near the surface of the skin. The ultrasound probe can also produce acoustic streaming, which refers to the large convective flows produced by ultrasound. This appears to aid in enhancing the rate of ISF extraction. More details in regards to ultrasound can be found in U.S Patent No. 6,234,990.

As depicted in FIG. 2, the particular sampling module 18 of system 10 is, however, an ISF sampling module that includes a penetration member 22 for penetrating a target site (TS) of body B and extracting an ISF sample, a launching mechanism 24 and at least one pressure ring 28. ISF sampling module 18 is adapted to provide a continuous or semi-continuous flow of ISF to analysis module 20 for the monitoring (e.g., concentration measurement) of an analyte (such as glucose) in the ISF sample.

During use of system 10, penetration member 22 is inserted into the target site (i.e., penetrates the target site) by operation of launching mechanism 24. For the extraction of an ISF sample from a user's skin layer, penetration member 22 can be inserted to a maximum insertion depth in the range of, for example, 15 mm to 3 mm. In addition, penetration member 22 can be configured to optimize extraction of an ISF sample in a continuous or semi-continuous manner. In this regard, penetration member 22 can include, for example, a 25 gauge, thin-wall stainless steel needle (not shown in FIGs. 1 or 2) with a bent tip, wherein a fulcrum for the tip bend is disposed between the needle's tip and the needle's heel. Suitable needles for use in penetration members according to the present invention are described in U.S. Patent Application Publication No. US 2003/0060784 A1 (U.S. Patent Application No. 10/185,605).

Launching mechanism 24 can optionally include a hub (not shown in FIGS. 1 or 2) surrounding penetration member 22. Such a hub is configured to control the insertion depth of penetration member 22 into the target site. Insertion depth control can be beneficial during the extraction of an ISF sample by preventing inadvertent lancing of blood capillaries, which are located relatively deep in a user's skin layer, and thereby eliminating a resultant fouling of an extracted ISF sample, clogging of the penetration member or clogging of an analysis module by blood. Controlling insertion depth can also serve to minimize pain and/or discomfort experienced by a user during use of system 10.

Such a hub can, in addition to controlling the insertion depth, be locked onto (integrated with) a pressure ring after launching of a penetration member and thus serve as an appendage of the pressure ring. Alternatively, the hub itself can be configured to serve both as an insertion depth control means and as a pressure ring following launch of the penetration member.

Although FIG. 2 depicts launching mechanism 24 as being included in sampling module 18; launching mechanism 24 can optionally be included in disposable cartridge 12 or in local controller module 14 of system 10. Furthermore, to simplify employment of system 10 by a user, sampling module 18 can be formed as an integral part of the analysis module 20.

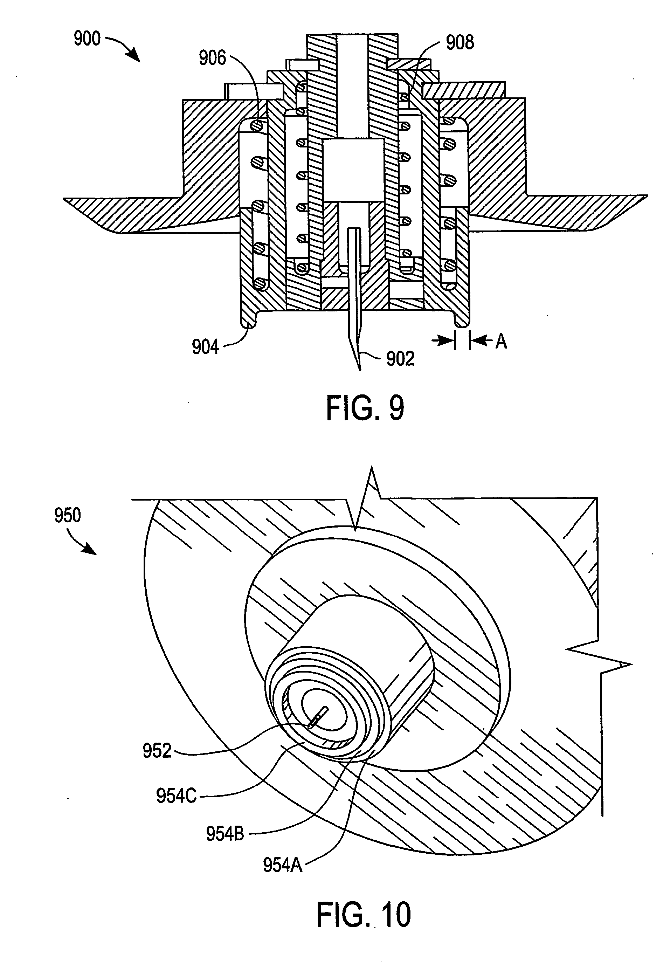

In order to facilitate the extraction of a bodily fluid (e.g., ISF) from the target site, penetration member 22 can be arranged concentrically within at least one pressure ring 28. Pressure ring(s) 28 can be of any suitable shape, including but not limited to, annular. In addition, pressure ring(s) 28 can be configured to apply an oscillating mechanical force (i.e., pressure) in the vicinity of the target site while the penetration member is residing in the user's skin layer. Such oscillation can be achieved through the use of a biasing element (not shown in FIGs. 1 or 2), such as a spring or a retention block. The structure and function of a pressure ring(s) in sampling modules (and ISF extraction devices) according to the present invention are described in more detail below with respect to FIGs. 9-12.

During use of system 10, pressure ring 28 is applied in the vicinity of the target site TS, prior to penetration of the target site by penetration member 22, in order to tension the user's skin layer. Such tension serves to stabilize the user's skin layer and prevent tenting thereof during penetration by the penetrating member. Alternatively, stabilization of the user's skin layer prior to penetration by the penetrating member can be achieved by a penetration depth control element (not shown) included in sampling module 18. Such a penetration depth control element rests or "floats" on the surface of the user's skin layer, and acts as a limiter for controlling penetration depth (also referred to as insertion depth). Examples of penetration depth control elements and their use are described in US publication no. 2005/0025839 (U.S. Patent Application No. 10/690,083). If desired, the penetration member can be launched coincidentally with application of the pressure ring(s) to the user's skin layer, thereby enabling a simplification of the launching mechanism.

Once penetration member 22 has been launched and has penetrated the target site TS, a needle (not shown in FIGs. 1 or 2) of penetration member 22 will reside, for example, at an insertion depth in the range of about 1.5 mm to 3 mm below the surface of the user's skin layer at the target site. The pressure ring(s) 28 applies/apply a force on the user's skin layer (indicated by the downward pointing arrows of FIG. 2) that pressurizes ISF in the vicinity of the target site. A sub-dermal pressure gradient induced by the pressure ring(s) 28 results/result in flow of ISF up the needle and through the sampling module to the analysis module (as indicated by the curved and upward pointing arrows of FIG. 2).

ISF flow through a penetration member's needle is subject to potential decay over time due to depletion of ISF near the target site and due to relaxation of the user's skin layer under the pressure ring(s) 28. However, in systems according to the present invention, pressure ring(s) 28 can be applied to the user's skin layer in an oscillating manner (e.g., with a predetermined pressure ring(s) cycling routine or with a pressure ring cycling routine that is controlled via ISF flow rate measurement and feedback) while the penetration member is residing in the user's skin layer in order to minimize ISF flow decay. In addition, during application of pressure in an oscillating manner, there can be time periods during which the pressure applied by the pressure ring(s) is varied or the local pressure gradient is removed and the net outflow of ISF from the user's skin layer is eliminated.

Furthermore, alternating the application of a plurality of pressure rings to the user's skin layer in the vicinity of the target site can serve to control the flow of ISF through the sampling and analysis modules and limit the time that any given portion of the user's skin layer is under pressure. By allowing a user's skin layer to recover, the application of pressure in an oscillating manner also reduces blemishes on the user's skin and a user's pain and/or discomfort. An additional beneficial effect of applying pressure ring(s) 28 in an oscillating manner is that ISF glucose lag (i.e., the difference between glucose concentration in a user's ISF and glucose concentration in a user's blood) is reduced.

Once apprised of the present disclosure, one skilled in the art can devise a variety of pressure ring cycling routines that serve to reduce ISF glucose lag, a user's pain/discomfort and/or the creation of persistent skin blemishes. For example, the pressure ring(s) 28 can be deployed (i.e., positioned such that pressure is applied to a user's skin layer in the vicinity of a target site) for a period of from 30 seconds to 3 hours and can then be retracted (i.e., positioned such that pressure is not being applied to the user's skin layer) for a period ranging from 30 seconds to 3 hours. Moreover, it has been determined that ISF glucose lag and a user's pain/discomfort are significantly reduced when the amount of time during which pressure is applied (i.e., the time period during which at least one pressure ring is deployed) is in the range of about 30 seconds to about 10 minutes and the amount of time during which pressure is released (i.e., the time period during which the at least one pressure ring is retracted) is in the range of about 5 minutes to 10 minutes. A particularly beneficial pressure ring cycle includes the application of pressure for one minute and the release of pressure for 10 minutes. Since different amounts of time are used for applying and releasing pressure, such a cycle is referred to as an asymmetric pressure ring cycle.

Pressure ring cycling routines can be devised such that the following concerns are balanced: (i) having the pressure ring(s) deployed for a time period that is sufficient to extract a desired volume of bodily fluid, (ii) inducing a physiological response that mitigates ISF glucose lag, and (iii) minimizing user discomfort and the creation of persistent blemishes. In addition, pressure ring cycling routines can also be devised to provide for semi-continuous analyte measurements that occur, for example, every 15 minutes.

Pressure ring(s) 28 can be formed of any suitable material known to those of skill in the art. For example, the pressure ring(s) 28 can be composed of a relatively rigid material, including, but not limited to, acrylonitrile butadiene styrene plastic material, injection moldable plastic material, polystyrene material, metal or combinations thereof. The pressure ring(s) 28 can also be composed of relatively resiliently deformable material, including, but not limited to, elastomeric materials, polymeric materials, polyurethane materials, latex materials, silicone materials or combinations thereof.

An interior opening defined by the pressure ring(s) 28 can be in any suitable shape, including but not limited to, circular, square, triangular, C-shape, U-shape, hexagonal, octagonal and crenellated shape.

When pressure ring(s) 28 is being employed to minimize ISF flow decay and/or control the flow of ISF through the sampling and analysis modules, penetration member 22 remains deployed in (i.e., residing in) the target site of the user's skin layer while the pressure ring(s) 28 is/are in use. However, when pressure ring(s) 28 are being employed to mitigate ISF glucose lag, the penetration member 22 can intermittently reside in the user's skin layer. Such intermittent residence of the penetration member 22 can occur either in or out of concert with the application of pressure by the pressure ring(s) 28.

In addition to the use of pressure ring(s) for mitigating ISF glucose lag, various embodiments of inventions according to the present invention can employ other means for mitigating ISF glucose lag, such as, for example, a chemical means (i.e., a lag mitigating chemical), ultrasound, mechanical means, heat, vacuum, electric potential, or a combination thereof. In general, such means for mitigating ISF glucose lag are hypothesized to increase the perfusion of blood and/or ISF in the vicinity of the means used for mitigating ISF glucose lag. By increasing the localized circulation of bodily fluid, this increases the equilibration rate of glucose between blood and ISF.

A chemical means may be used to mitigate glucose lag. Such chemical means involve applying a lag mitigating chemical to a target site (e.g., a the user's skin layer) to enhance circulation. Exemplary and non-limiting chemical compounds which can perform this function are capsaicin, histamine, natural bile salts, sodium cholate, sodium dodecyl sulfate, sodium deoxycholate, taurodeoxycholate, sodium glucocholate, or a combination thereof. In addition, all skin permeation enhancers and combinations thereof which are described and referenced within U.S. Patent No.'s 6,251,083 and 5,139,023 are suitable candidates for use. The chemical means may be incorporated into an emulsion or gel to allow for a simple and direct application of the chemical means. In addition, an absorbent material such as fleece may be used to facilitate the amount of chemical means which is applied.

Another means for mitigating ISF glucose lag is to use ultrasound. Ultrasound lag mitigating techniques involve the application of ultrasound to a target site by placing an ultrasound probe adjacent to the target site (e.g., a user's skin layer). Applying a first pre-determined amount of ultrasound to the target site causes localized heating which in turn helps mitigate ISF glucose lag. In certain embodiments, after mitigating glucose lag, the ultrasound probe can then apply a second pre-determined amount of ultrasound, which is greater than the first pre-determined amount, to facilitate the extraction of ISF. In such an embodiment, the ultrasound probe performs both the function of mitigating glucose lag and extracting ISF. Further details regarding ultrasound techniques are in U.S. Patent No.'s 5,231,975 and 5,458,140.

A further means (technique) for mitigating ISF glucose lag is non-oscillatory mechanical manipulation. Such mechanical manipulation can include pulling or pinching a target site, adhesives which bring about target site stretching by means of pulling, and devices for imparting vibration to the user's skin layer (i.e. piezoelectric transducer). Mechanical means for manipulating target sites are described in U.S. Patent No.'s 6,332,871 and 6,319,210.

Yet a further means for mitigating glucose lag is the use of heat. In such means, a heating probe (e.g., a resistive heater) can be applied to a target site (such as a user's skin) to enhance the circulation of bodily fluids. Alternatively" an infra-red (IR) source can be employed as a heat source. In such embodiments, a temperature probe can be used to ensure that an appropriate amount of heat is applied to the user's skin layer such that the treatment is comfortable to the user and that the duration of heat treatment is a relatively short time interval (i.e. less than 5 minutes). In general, the applied heat must be greater than 37 °C, but not too high such that the user's skin layer will burn. Details regarding the application of heat to a target site are in U.S. Patent No.'s 6,240,306 and 6,155,992.

Still yet another means for mitigating ISF glucose lag is the use of vacuum. For example, vacuum can help stretch a target site (such as a user's skin layer), which in turn can aid in mitigating ISF glucose lag. In addition, the vacuum provides a negative pressure source which can facilitates ISF extraction from the target site. The application of vacuum to target sites is described in U.S. Patent No.'s 6,155,992.

Still yet further means for mitigating ISF glucose lag is the use of an electric potential. In such a circumstance, a pair of electrodes is, for example, used to apply a current to a target site (such as a user's skin layer). The current stimulates nerve cells and tissues in a way that enhances circulation and mitigates ISF glucose lag.

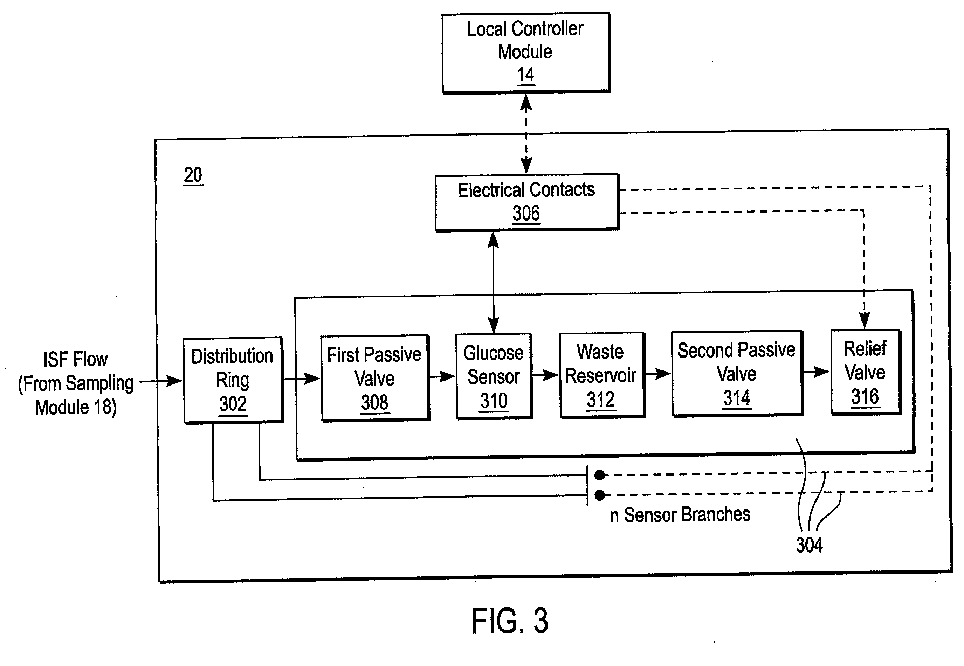

Referring to FIG. 3, analysis module 20 of system 10 includes a distribution ring 302, a plurality of micro-fluidic networks 304 and a plurality of electrical contacts 306. Each of micro-fluidic networks 302 includes a first passive valve 308, a glucose sensor 310, a waste reservoir 312, a second passive valve 314 and a relief valve 316. Micro-fluid networks 304 include channels with a cross-sectional dimension in the range of, for example, 30 to 500 micrometers. For monitoring (e.g., measuring) glucose in a flowing ISF sample, a plurality (n) of essentially identical micro-fluidic networks 304 (also referred to as sensor branches 304) can be included in analysis module 20. Distribution ring 302, first passive valve 308, waste reservoir 312, second passive valve 314 and a relief valve 316 are configured to control ISF flow through analysis module 20.

Any suitable glucose sensor known to those of skill in the art can be employed in analysis modules according to the present invention. Glucose sensor 310 can contain, for example, a redox reagent system including an enzyme and a redox active compound(s) or mediator(s). A variety of different mediators are known in the art, such as ferricyanide, phenazine ethosulphate, phenazine methosulfate, phenylenediamine, 1-methoxy-phenazine methosulfate, 2,6-dimethyl-1,4-benzoquinone, 2,5-dichloro-1,4-benzoquinone, ferrocene derivatives, osmium bipyridyl complexes, and ruthenium complexes. Suitable enzymes for the assay of glucose in whole blood include, but are not limited to, glucose oxidase and dehydrogenase (both NAD and PQQ based). Other substances that may be present in the redox reagent system include buffering agents (e.g., citraconate, citrate, malic, maleic, and phosphate buffers); divalent cations (e.g., calcium chloride, and magnesium chloride); surfactants (e.g., Triton, Macol, Tetronic, Silwet, Zonyl, and Pluronic); and stabilizing agents (e.g., albumin, sucrose, trehalose, mannitol and lactose).

In the circumstance that glucose sensor 310 is an electro-chemical based glucose sensor, glucose sensor 310 can produce an electrical current signal in response to the presence of glucose in an ISF sample. Local controller module 14 can then receive the electrical current signal (via electrical contacts 306) and convert it into ISF glucose concentration.

System 10 can be employed for the continuous and/or semi-continuous measurement (monitoring) of glucose in an ISF sample for a period of eight hours or more. However, conventional glucose sensors that can be economically mass-produced provide an accurate measurement signal for a lifetime of only about one hour. In order to overcome this problem of limited sensor lifetime, a plurality of micro-fluid networks 304, each containing an identical glucose sensor 310, are provided in analysis module 20. Each of these glucose sensors is employed in a consecutive manner to provide continuous and/or semi-continuous monitoring for a period of more than one hour.

The consecutive use of identical glucose sensors (each for a limited period of time, such as one hour) enables a continuous or semi-continuous measurement of glucose. The consecutive use of identical glucose sensors can be implemented by guiding an incoming flow of ISF from a sampling module towards a glucose sensor 310 for a period of time, followed by interrupting the ISF flow to that glucose sensor and switching the ISF flow to another glucose sensor. This consecutive use of glucose sensors can be repeated until each glucose sensor included in an analysis module has been used.

The switching of the ISF flow to consecutive glucose sensors can be accomplished, for example, by the following procedure. Upon initialization of analysis module 20, an ISF sample from sampling module 18 is distributed via distribution ring 302 to "n" sensor branches 304. However, the flow of ISF is halted at an inlet end of each sensor branch by the first passive valve 308 of each sensor branch. To start the measurement of glucose, a selected sensor branch is activated by opening the relief valve 316 of that sensor branch. The process of opening a selected relief valve can be electrically controlled by local controller module 14, which communicates with analysis module 20 via electrical contacts 306. Upon opening of a relief valve 316, gas (e.g., air) that is initially present in the sensor branch 304 (which is hermetically sealed) escapes at an outlet end of the sensor branch 304, and, as a result, ISF will flow into that sensor branch 304. As the relief valves 316 of the other sensor branches 304 remain closed, the ISF is allowed to flow only into the selected sensor branch 304.

The pressure of the ISF is sufficiently large to breach first passive valve 308 and will, therefore, flow towards glucose sensor 310. A measurement signal is subsequently created by glucose sensor 310 and communicated electronically via electrical contacts 306 to the local controller module 14 (as depicted by the dashed arrows in FIG. 3). ISF continues flowing and enters waste reservoir 312, the volume of which is predetermined such that it can contain an amount of ISF equivalent to that needed through the glucose sensor's lifetime. For example, at the average flow rate of about 50 nanoliters per minute and a glucose sensor lifetime of one hour, the volume of waste reservoir 312 would be approximately 3 microliters. A second passive valve 314 is located at the end of the waste reservoir 312. The second passive valve 314 is configured to stop the flow of ISF.

The procedure then continues by opening of a relief valve 316 of another sensor branch 304. Upon selectively opening this relief valve 316 (which can be accomplished via communication by the local controller module 14), ISF will flow into the corresponding sensor branch 304 after breaching the first passive valve 308 located in that sensor branch. Thereafter, the glucose sensor 310 of that sensor branch will provide a measurement signal to analysis module 20.

This procedure is repeated until all sensor branches 304 of analysis module 20 have been used. For a system to provide about eight hours of continuous glucose monitoring, about eight sensor branches 304 will be required in analysis module 20. It will be appreciated by those skilled in the art, however, that the analysis module 20 of disposable cartridge 12 is not limited to eight sensor branches and that, therefore, the system can be designed to measure ISF glucose levels for longer (or even shorter) than eight hours.

It should be noted that analysis module 18 has thus far been described as being external to the body B. In an alternative embodiment of a system according to the present invention, a sampling module is not employed. However, a portion of analysis module 18 (which includes, for example, a glucose sensor)is at least partially implanted into body B (for example, into a subcutaneous layer of body B). Suitable continuous glucose sensors include those described in U.S. Patent No.'s 6,514,718; 6,329,161; 6,702,857 and 6,558,321.

Such glucose sensors can employ an enzyme, such as glucose oxidase or glucose dehydrogenase, co-immobilized with an osmium redox polymer onto a working electrode. A bifunctional crosslinking reagent such as an epoxide or aziridine may be used to co-immobilize the enzyme and polymer to the electrode surface. Such a glucose sensor can measure glucose without the addition of any freely diffusing reagents and can transduce a glucose concentration into a proportional current level or charge.

Other glucose sensors can employ an enzyme such as glucose oxidase immobilized onto a working electrode. Typically, a bifunctional crosslinking reagent such as glutaraldehyde is used to immobilize the enzyme to the working electrode. In such a glucose sensor, oxygen is converted to hydrogen peroxide such that the hydrogen peroxide concentration is proportional to the glucose concentration. The hydrogen peroxide is then oxidized at the working electrode so that a current magnitude can be ascertained for determining the level of the glucose present in ISF.

Yet another glucose sensor employs a modified bead (such as a latex bead) that can be implanted into the subcutaneous layer and which uses fluorescence resonance energy transfer (FRET) technology to monitor glucose.

Additional details regarding such glucose monitors are in U.S. Patent No.'s 6,232,130 and 6,040,194.

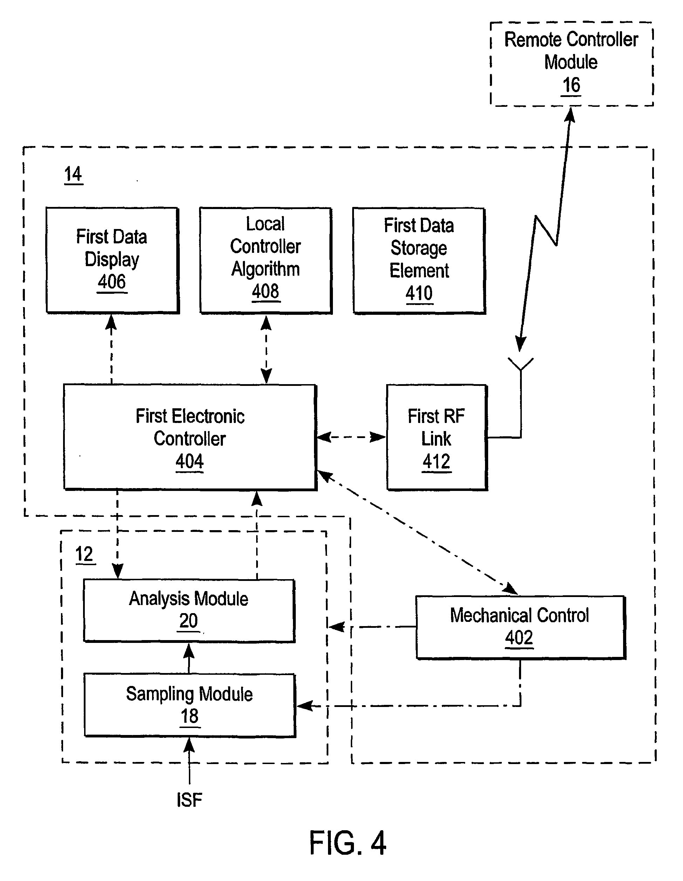

Local controller module 14 is depicted in simplified block form in FIG. 4. Local controller module 14 includes a mechanical controller 402, a first electronic controller 404, a first data display 406, a local controller algorithm 408, a first data storage element 410 and a first RF link 412.

Local controller module 14 is configured such that it can be electrically and mechanically coupled to disposable cartridge 12. The mechanical coupling provides for disposable cartridge 12 to be removably attached to (e.g., inserted into) local controller module 14. Local controller module 14 and disposable cartridge 12 are configured such that they can be attached to the skin of a user by, for example, a strap, in a manner which secures the combination of the disposable cartridge 12 and local controller module 14 onto the user's skin.

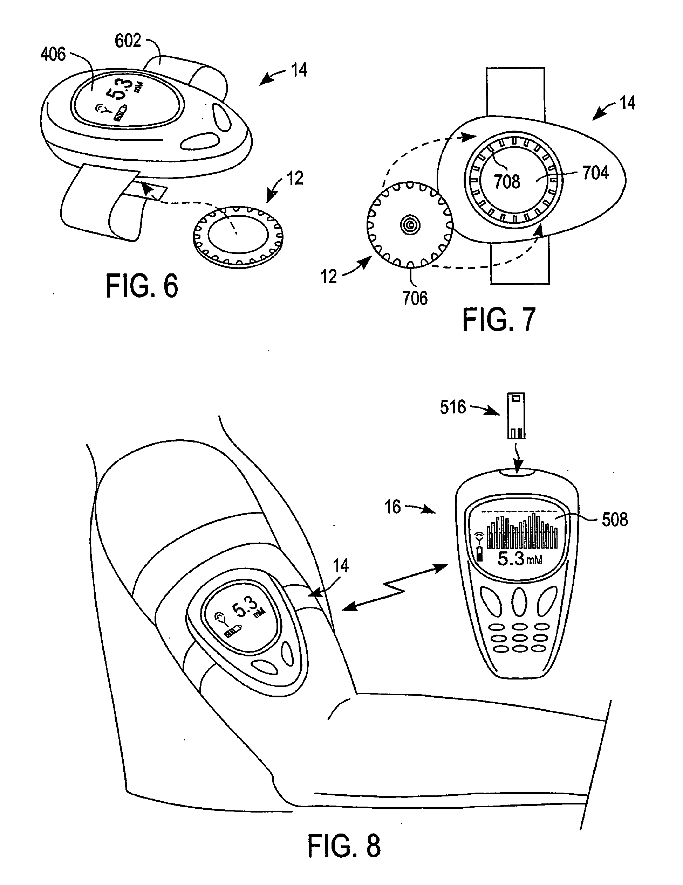

During use of system 10, first electronic controller 404 controls the measurement cycle of the analysis module 20, as described above. Communication between local controller module 14 and disposable cartridge 12 takes place via electrical contacts 306 of analysis module 20 (see FIG. 3). Electrical contacts 306 can be contacted by contact pins 708 (see FIG. 7) of the local controller module 14. Electrical signals are sent by the local controller module 14 to analysis module 20 to, for example, selectively open relief valves 316. Electrical signals representing the glucose concentration of an ISF sample are then sent by the analysis module to the local controller module. First electronic controller 404 interprets these signals by using the local controller algorithm 408 and displays measurement data on a first data display 406 (which is readable by the user). In addition, measurement data (e.g., ISF glucose concentration data) can be stored in first data storage element 409.

Prior to use, an unused disposable cartridge 12 is inserted into local controller module 14. This insertion provides for electrical communication between disposable cartridge 12 and local controller module 14. A mechanical controller 402 in the local controller module 14 securely holds the disposable cartridge 12 in place during use of system 10.

After attachment of a local controller module and disposable cartridge combination to the skin of the user, and upon receiving an activation signal from the user, a measurement cycle is initiated by first electronic controller 404. Upon such initiation, penetration member 22 is launched into the user's skin layer to start ISF sampling. The launching can be initiated either by first electronic controller 404 or by mechanical interaction by the user.

First RF link 412 of local controller module 14 is configured to provide bi-directional communication between the local controller module and a remote controller module 16, as depicted by the jagged arrows of FIGs. 1 and 4. The local controller module incorporates a visual indicator (e.g., a multicolor LED) indicating the current status of the system.

Local controller module 14 is configured to receive and store measurement data from, and to interactively communicate with, disposable cartridge 12. For example, local controller module 14 can be configured to convert a measurement signal from analysis module 20 into an ISF or blood glucose concentration value.

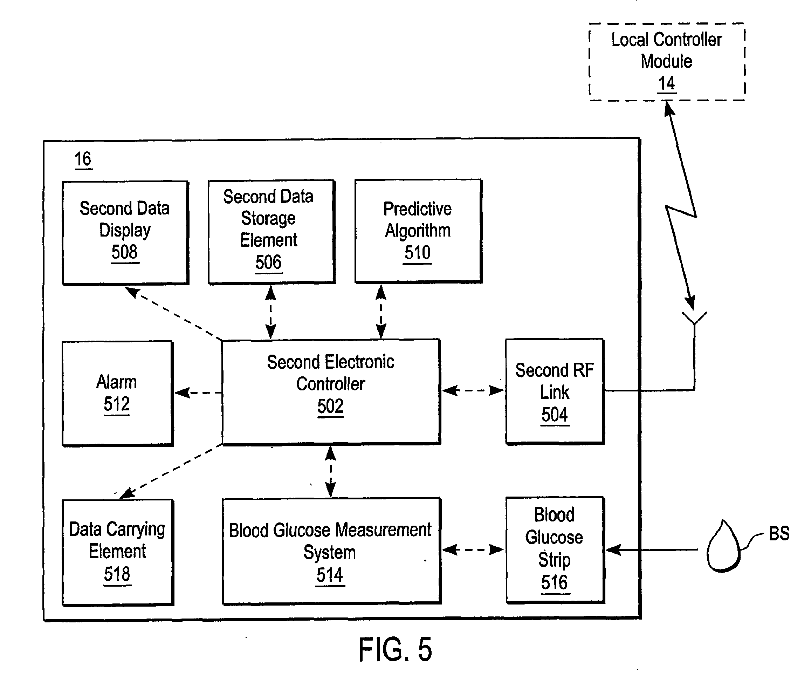

FIG. 5 shows a simplified block diagram depicting remote controller module 16 of system 10. Remote controller module 16 includes a second electronic controller 502, a second RF link 504, a second data storage element 506, a second data display 508, a predictive algorithm 510, an alarm 512, a blood glucose measurement system (adapted to measure blood glucose utilizing blood glucose strip 516) and a data carrying element 518.

Second electronic controller 502 is adapted to control various components of remote controller module 16. Second RF link 504 is configured for bi-directional communication with the local controller module 14 (e.g., second RF link 504 can receive ISF glucose concentration related data from local controller module 14). Data received via second RF link 504 can be validated and verified by second electronic controller 502. Furthermore, the data so received can also be processed and analyzed by second electronic controller 502 and stored in second data storage element 506 for future use (e.g., future data retrieval by a user or for use in predictive algorithm 510). Second data display 508 of remote controller module 16 can be, for example, a graphic LCD display configured to present measurement data in a convenient format to a user and to present an easy to use interface for further data management.

The local controller module 14 is adapted to communicate via second RF link 504 to a remote controller module 16. Functions of remote controller module 16 include the displaying, storing and processing of glucose measurement data in a presentable and convenient format for the user. Remote controller module 16 can also provide an (audible, visual and/or vibratory) alarm via alarm 512 for warning the user of deleterious glucose concentrations. A further function of remote controller module 16 is to measure a user's blood glucose concentration using blood glucose measurement system 514 and a single use blood glucose measurement strip 516. Blood glucose values measured by blood glucose measurement system 514 can be used to verify blood glucose values calculated by predictive algorithm 510. Remote controller module 16 can also be configured to provide for user specific data (e.g., event tags, state of mind and medical data) to be entered and parsed.

Remote controller module 16 is configured as a portable unit and to communicate with local controller module 14 (e.g., to receiving glucose measurement data from local controller module 14). Remote controller module 16, therefore, provides a user with a simple and convenient platform for managing glucose monitoring-related data (e.g., storing, displaying and processing of glucose monitoring-related data) and can be used to fine tune therapy (i.e., insulin administration). Functions of the remote controller module 16 can include the gathering, storing and processing of ISF glucose data and the display of the blood glucose value calculated from ISF glucose data. By incorporating such functions in remote controller module 16, rather than local controller module 14, the size and complexity of local controller module 14 are reduced. However, if desired, the remote controller module functions described above can be alternatively performed by the local controller module.

In order to facilitate a measurement of the blood glucose level in a blood sample (BS), blood glucose measurement system 514 is provided as an integral part of the remote controller module 16. The blood glucose measurement system 514 makes a measurement with a blood glucose strip 516, on which a blood sample (e.g., a drop of blood) has been placed. The resulting blood glucose measurement can be compared to glucose values calculated by predictive algorithm 510. '

Remote controller module 16 can optionally incorporate a communication port, such as a serial communication port (not shown in FIG. 5). Suitable communication ports are known in the art, for example, an RS232 (IEEE standard) and a Universal Serial Bus. Such communication ports can be readily adopted for exporting stored data to an external data management system. Remote controller module 16 also incorporates a programmable memory portion (not shown in FIG. 5), such as a reprogrammable flash memory portion, that can be programmed via a communication port. A purpose of such a memory portion is to facilitate updates of an operating system and/or other software element of the remote controller module via communication through the communication port.

The remote controller module 16 can further include a communication slot (not shown) for receiving a data carrying element 518 and communicating therewith. Data carrying element 518 can be any suitable data carrying element known in the art, such as a 'SIM' data carrying element, also known as "smart-chip."

Data carrying element 518 can be provided with a disposable cartridge 12 and can contain disposable cartridge production lot specific data, such as calibration data and lot identification number. The remote controller module 16 can read the data contained on data carrying element 518 and such data can be employed in the interpretation of the ISF glucose data received from the local controller module 14. Alternatively, the data on data carrying element 518 can be communicated to the local controller module 14 via second RF link 504 and can be used in data analysis performed by the local controller module 14.

The second electronic controller 502 of remote controller module 16 is configured to interpret data, as well as to perform various algorithms. One particular algorithm is predictive algorithm 510 for predicting near future (within 0.5-1 hour) glucose levels. As there is a time difference ("lag time") between changes of glucose concentration in the blood of the user and the corresponding change of glucose concentration in the ISF of the user, predictive algorithm 510 uses a series of mathematical operations performed on the stored measurement data to take into account user specific parameters reflecting individual lag time relationships. The outcome of the predictive algorithm 510 is an estimation of the blood glucose level based on the ISF glucose level. If the predictive algorithm 510 predicts low glucose levels, a signal can be raised and alarm 512 activated to warn the user of a predicted physiological event such as hypoglycemia or risk of coma. As will be appreciated by those skilled in the art, the alarm 512 may be comprised of any suitable signal including an audible, visual or vibratory signal, warning either the user directly or the user's health care provider. An audible signal is preferred, as it will wake up a sleeping user encountering a hypoglycemic event.

The difference between an ISF glucose value (concentration) at any given moment in time and a blood glucose value (concentration) at the same moment in time is referred to as the ISF glucose lag. ISF glucose lag can be conceivably attributed to both physiological and mechanical sources. The physiological source of lag in ISF glucose is related to the time it takes for glucose to diffuse between the blood and interstices of a user's skin layer. The mechanical source of lag is related to the method and device used to obtain an ISF sample.

Embodiments of devices, systems and methods according to the present invention mitigate (reduce or minimize) ISF glucose lag due to physiological sources by applying and releasing pressure to a user's skin layer in an oscillating manner that enhances blood flow to a target area of the user's skin layer. ISF extraction devices that include pressure ring(s) according to the present invention (as described in detail below) apply and release pressure in this manner. Another approach to account for lag in ISF glucose is to employ an algorithm (e.g., predictive algorithm 510) that predicts blood glucose concentration based on measured ISF glucose concentrations.

Predictive algorithm 510 can, for example, take the general form:

$$\text{Predicted blood glucose} = f(\text{ISF}_i^k, \text{rate}_j, \text{ma}_n\text{rate}_m^p, \text{interaction terms})$$

where:

i is an integer of value between 0 and 3;
j, n, and m are integers of value between 1 and 3;
k and p are integers of value 1 or 2;
$\text{ISF}_i$ is a measured ISF glucose value with the subscript (i) indicating which ISF value is being referred to, i.e., 0 = current value, 1 = one value back, 2 = two values back, etc.;

rate$_j$ is the rate of change between adjacent ISF values with the subscript (i) referring to which adjacent ISF values are used to calculate the rate, i.e., 1 = rate between current ISF value and the previous ISF value, 2= rate between the ISF values one previous and two previous relative to the current ISF value, etc.; and

ma$_n$rate$_m$ is the moving average rate between adjacent averages of groupings of ISF values, with the subscripts (n) and (m) referring to (n) the number of ISF values included in the moving average and (m) the time position of the moving adjacent average values relative to the current values as follows.

The general form of the predictive algorithm is a linear combination of all allowed terms and possible cross terms, with coefficients for the terms and cross terms determined through regression analysis of measured ISF values and blood glucose values at the time of the ISF sample acquisition. Further details regarding predictive algorithms suitable for use in systems according to the present invention are included in US publication no. 2004/0253736 (U.S. Patent Application No. 10/652,464).

As will also be appreciated by those skilled in the art, the outcome of the predictive algorithm can be used to control medical devices such as insulin delivery pumps. A typical example of a parameter that can be determined based on the algorithm outcome is the volume of a bolus of insulin to be administered to a user at a particular point in time.

The combination of local controller module 14 and disposable cartridge 12 can be configured to be worn on the skin of a user in order to simplify sampling and monitoring of ISF extracted from the user's skin layer (see FIGs. 6-8).

During use of the system embodiment of FIGs. 1-10, disposable cartridge 12 is located within and controlled by local controller module 14. In addition, the combination of disposable cartridge 12 and local controller module 14 is configured to be worn by a user, preferably on the upper part of the user's arm or forearm. The local controller module 14 is in electrical communication with the disposable cartridge 12 for purposes of measurement control and for receiving measurement data from the analysis module.

Referring to FIG. 6, local controller module 14 includes a first data display 406 and a pair of straps 602 for attachment of the local controller module 14 to the arm of a user. FIG. 6 also depicts disposable cartridge 12 prior to insertion into local controller module 14.

FIG. 7 shows a bottom view of the local controller module 14 prior to the insertion of the disposable cartridge 12 into an insertion cavity 704 provided in local controller module 14. The disposable cartridge 12 and local controller module 14 are configured such that disposable cartridge 12 is secured within the insertion cavity 704 by mechanical force. In addition, the local controller module 14 and the disposable cartridge 12 are in electrical communication via a set of molded contact pads 706 that are provided on disposable cartridge 12. These molded contact pads 706 are in registration with a set of contact pins 708 provided within the insertion cavity 704 of the local controller module 14 when the disposable cartridge is inserted into insertion cavity 704.

FIG. 8 shows the local controller module 14 after insertion of the disposable cartridge 12 into local controller module 14 and attachment of the combination of the disposable cartridge and local controller module onto the arm of a user. FIG. 8 also depicts a remote controller module 16 located within RF communication range of the local controller module 14.

FIG. 9 is a cross-sectional side view of an interstitial fluid (ISF) extraction device 900 according to an exemplary embodiment of the present invention. ISF extraction device 900 includes a penetration member 902, a pressure ring 904, a first biasing member 906 (i.e., a first spring) and a second biasing member 908 (namely, a second spring).

Penetration member 902 is configured for penetration of a user's skin layer at a target site and for the subsequent extraction of ISF therefrom. Penetration member 902 is also configured to remain in (reside in) the user's skin layer during the extraction of ISF therefrom. Penetration member 902 can, for example, remain in the user's skin layer for more than one hour, thus allowing a continuous or semi-continuous extraction of ISF. Once apprised of the present disclosure, one skilled in the art will recognize that the penetration member can reside in the user's skin layer for an extended period of time of 8 hours or more.

Pressure ring 904 is configured to oscillate between a deployed state and a retracted state. When pressure ring 904 is in the deployed state, it applies pressure to the user's skin layer surrounding the target site, while the penetration member is residing in the user's skin layer in order to (i) facilitate the extraction of ISF from the user's skin layer and (ii) control the flow of ISF through ISF extraction device 900 to, for example, an analysis module as described above. When pressure ring 904 is in a retracted state, it applies either a minimal pressure or no pressure to the user's skin layer surrounding the target site. Since pressure ring 904 can be oscillated between a deployed state and a retracted state, the time that any given portion of a user's skin layer is under pressure can be controlled, thereby providing for the user's skin layer to recover and reducing pain and blemishes.

Pressure ring 904 typically has, for example, an outside diameter in the range of 2.03 mm (0.08 inches) to 14.2mm (0.56 inches) and a wall thickness (depicted as dimension "A" in FIG. 9) in the range of 0.51mm (0.02 inches) to 1.02mm (0.04 inches).

Penetration member 902 could be configured to move independently of pressure ring 904, although that is outside the scope of the present invention. According to the invention, the penetration member 902 is fixed with respect to pressure ring 904. In the circumstance that penetration member 902 is fixed with respect to pressure ring 904, penetration member 902 will move along with pressure ring 904. However, frictional forces between portions of a target site (e.g., skin of a target site) and penetration member 902 can provide for the target site to assume a "tent" configuration and for penetration member 902 to remain residing within the target site despite the penetration member moving along with the retraction of the pressure ring. In this regard, a benefit of having the penetration member fixed with respect to the pressure ring is simplicity of design.

First biasing element 906 is configured to urge pressure ring 904 against the user's skin layer (i.e., to place pressure ring 904 into a deployed state) and to retract pressure ring 904. Second biasing element 908 is configured to launch the penetration member 902 such that the penetration member penetrates the target site.

The pressure (force) applied against a user's skin layer by the pressure ring(s) can be, for example, in the range of from about 6.90 to 1034 kPa (1 to 150 pounds per square inch) (calculated as force per cross-sectional pressure ring area). In this regard, a pressure of approximately 345 kPa (50 PSI) has been determined to be beneficial with respect to providing adequate ISF flow while minimizing user pain/discomfort.

In the embodiment of FIG. 9, penetration member 902 is partially housed in a recess of the oscillating pressure ring 904, the depth of the recess determining the maximum penetration depth of the penetration member 902.

During use of ISF extraction device 900, the oscillating pressure ring 904 can be deployed for stabilizing the user's skin layer and to isolate and pressurize a region of the target area and thus to provide a net positive pressure to promote flow of ISF through penetration member 902.

If desired, ISF extraction device 900 can contain a penetration depth control element (not shown) for limiting and controlling the depth of needle penetration during lancing. Examples of suitable penetration depth control elements and their use are described in US publication no. 2005/00 85 839 U.S. Patent Application No. 10/690,083).

During use of ISF extraction device 900, a system that includes ISF extraction device 900 is placed against a user's skin layer with the pressure ring 904 facing the skin (see, for example, FIG. 8). The pressure ring 904 is urged against the skin to create a bulge. The bulge is then penetrated (e.g., lanced) by the penetration member 902. An ISF sample is subsequently extracted from the bulge while the penetration member 902 remains totally or partially within the skin.

The flow rate of the ISF sample being extracted is initially relatively high but typically declines over time. After a period in the range of 3 seconds to 3 hours, pressure ring 904 can be retracted to allow the skin to recover for a period of about 3 seconds to 3 hours. Pressure ring 904 can then be re-deployed for a period in the range of about 3 seconds to about 3 hours and retracted for about 3 seconds to 3 hours. This process of deploying and retracting pressure ring 904 proceeds until ISF extraction is discontinued. The deployment and retraction cycles are preferably asymmetric in that different periods of time are used for each cycle.

As described herein, pressure ring(s) (e.g., pressure ring 904 of FIG. 9) employed in embodiments of the present invention can be employed to mitigate (i.e., reduce) ISF glucose lag. It is hypothesized, without being bound, that such mitigation is a result of increased perfusion in the vicinity of a site from which an ISF sample is extracted or within which an analysis module is at least partially implanted. If desired, other suitable means for increasing perfusion, and thus mitigating ISF lag, can be combined with such pressure ring(s). For example, pressure ring 904 of FIG. 9 can be heated to increase perfusion. Such heating can be accomplished, for example, by passing an electric current through a resistive material embedded in pressure ring 904 or by circulating a heated fluid through a cavity within pressure ring 904. Suitable chemical-based means for increasing perfusion (and thus decreasing ISF glucose lag) include, for example, the application of topical vasodilators (e.g., histamine) in the vicinity of a site from which an ISF sample is extracted or within which an analysis module is at least partially implanted. Furthermore, an ultrasound transducer-based device configured for increasing perfusion can be incorporated into pressure ring 904 and/or electrical stimuli-based device configured for increasing perfusion can be incorporated into pressure ring 904.

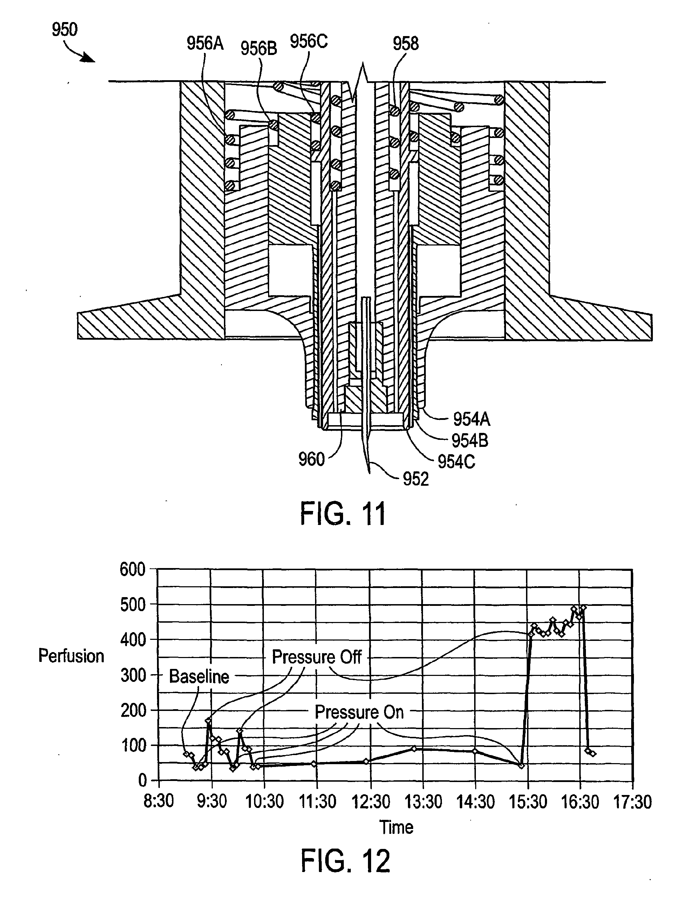

FIGs. 10 and 11 are cross sectional and perspective views, respectively, of an ISF extraction device 950 according to another exemplary embodiment of the present invention. ISF extraction device 950 includes a penetration member 952 and a plurality of concentrically arranged pressure rings 954A, 954B and 954C. ISF extraction device 950 also includes a plurality of first biasing elements 956A, 956B and 956C for urging the pressure rings 954A, 954B and 956C, respectively, toward and against a user's skin layer, a second biasing element 958 for launching the penetration member 952, and a penetration depth control element 960. If desired, penetration depth control element 960 can be integrated with pressure ring 954C to form an integrated penetration depth control and pressure ring element.

During use, ISF extraction device 950 is positioned such that pressure rings 954A, 954B and 954C are facing a user's skin layer. This can be accomplished, for example, by employing ISF extraction device 950 in a sampling module of a system for extracting bodily fluid as described above and placing the system against the user's skin layer.

Pressure ring 954A is then urged against the user's skin layer by biasing element 956A, thereby creating a bulge in the user's skin layer that will subsequently be lanced (i.e., penetrated) by penetration member 952. While pressure ring 954A is in use (i.e., deployed), pressure ring 954B and pressure ring 954C can be maintained in a retracted position by biasing elements 956B and 956C, respectively.

ISF can be extracted from the bulge formed in user's skin layer while the penetration member 952 resides totally or partially within the user's skin layer. After about 3 seconds to 3 hours, the pressure ring 954A can be retracted to allow the user's skin layer to recover for a time period in the range of about 3 seconds to 3 hours. After retracting the pressure ring 954A, pressure ring 954B can be deployed to apply pressure on the user's skin layer. While pressure ring 954B is in use (i.e., deployed), pressure ring 954A and pressure ring 954C can be maintained in a retracted position by biasing elements 956A and 956C, respectively. After a time period of about 3 seconds to 3 hours, pressure ring 954B can be retracted for a time period in the range of 3 seconds to 3 hours, followed by the deployment of pressure ring 954C. Pressure ring 954C maintains pressure on the user's skin layer for a time period in the range of 3 seconds to 3 hours and is then retracted for a time period in the range of 3 seconds to 3 hours. While pressure ring 954C is in use (i.e., deployed), pressure ring 954A and pressure ring 954B can be maintained in a retracted position by biasing elements 956A and 956B, respectively. This process of cycling between deployment and retraction of pressure rings 954A, 954B and 954C can proceeds until fluid extraction has ended. As with the embodiment shown in FIG. 9, the deployment and retraction cycles in the multiple pressure ring embodiment of FIGs. 10 and 11 are preferably asymmetric in that different periods of time are used for each cycle.

Those skilled in the art will also recognize that a plurality of pressure rings in ISF extraction devices according to the present invention can be deployed in any order and that one is not limited to the deployment and retraction sequence described above. For example, a sequence can be used in which pressure ring 954B or 954C is applied before pressure ring 954A. Further, more than one pressure ring can be deployed simultaneously. For example, the embodiment shown in FIGs. 10 and 11 can deploy all three pressure rings simultaneously such that the pressure rings function as a single pressure ring.

For the embodiment shown in FIGs. 10 and 11, the pressure applied against the user's skin can, for example, range from about 0.69 to 1034 kPa (0.1 to 150 pounds per square inch (PSI)) for each of the plurality of pressure rings. Furthermore, one skilled in the art will recognize that embodiments according to the present invention can employ pressure rings that provide a constant force against a target site (for example, a force of approximately 0-28 N (2 lbs)) during operation or a constant pressure (for example, a pressure of 138 to 207 kPa (20 to 30 pounds per square-inch)) during during operation. Optionally, the pressure or force can be varied within or between pressure application cycles. For example, the pressure can be varied from 138 to 207 kPa (20-30 pounds) within a 1 minute extraction cycle.

The pressure rings 954A, 954B and 954C can have, for example, outer diameters of in the range of 2-00 to 14-2 mm (0.08 to 0.560 inches) 2-54 to 22-9 nm (0.1 to 0.9 inches) and 4-07 to 24-4 nm (0.16 to 0.96 inches), respectively. The wall thickness of each pressure ring can be, for example, in the range of 0-51 to 1-02 mm (0.02 to 0.04 inches).

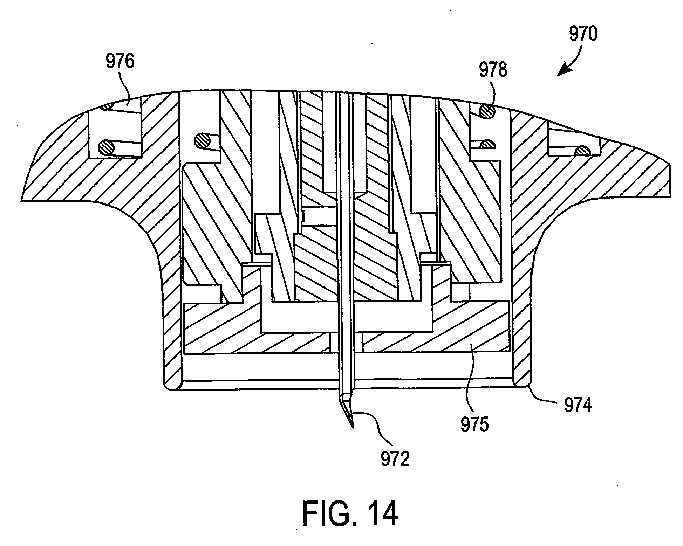

An inner-most pressure ring of extraction devices according to an alternative embodiment of the present invention can, if desired, be a flat ring (see FIG. 14 for the purpose of keeping the needle in the user's skin layer while applying negligible pressure to keep blood flowing to the area. FIG. 14 shows a cross-sectional side view of a portion of an interstitial fluid (ISF) extraction device 970 according to an alternative exemplary embodiment of the present invention. ISF extraction device 970 includes a penetration member 972, a pressure ring 974, a flat pressure ring 975, a first biasing member 976 (i.e., a first spring) for biasing the pressure ring 974 and a second biasing member 978 (namely, a second spring) for biasing the flat pressure ring.

In this alternate embodiment, the flat pressure ring surrounds the needle (i.e., the penetration member 972) and contains a hole of sufficient size to just allow the needle to pass through. The flat pressure ring preferably has a diameter of 0-51 to 14-2 mm (0.02 to 0.56 inches)

Inclusion of at least one pressure ring in extraction devices according to the present invention provides a number of benefits. First, oscillating the pressure ring(s) between a deployed and retracted state serves to mitigate (i.e., reduce) ISF glucose lag. Upon retraction of the pressure ring(s), pressure on the user's skin layer is released, and the user's body reacts by increasing blood perfusion to the target site. This phenomenon is known as reactive hyperemia and is hypothesized to be a mechanism by which ISF is beneficially replenished in the target site by oscillation of the pressure ring(s). Such a replenishment of ISF helps in mitigating the lag between the ISF glucose and whole blood glucose values.

Another benefit of ISF extraction devices according to the present invention is that oscillation of the pressure ring(s) allows the skin under the pressure ring(s) to recover, thus reducing a user's pain, discomfort and the creation of persistent blemishes.

Moreover, extraction devices with a plurality of pressure rings (e.g., the embodiment of FIGs.10 and 11) can be used.with at least one pressure ring permanently deployed to facilitate ISF collection while the other pressure rings are oscillated between deployed and retracted states so that different areas of the user's skin layer are under pressure at any given time. Such combination of permanently deployed pressure ring(s) and oscillated pressure ring(s) further aids in reducing a user's pain/discomfort.

Still another benefit of ISF extraction devices according to the present embodiment is that the pressure ring(s) can be used to control the conditions under which a glucose measurement of an extracted ISF sample is conducted. For example, an electrochemical glucose sensor is more accurate and precise if the ISF sample flow rate past the glucose sensor is constant or static. The pressure ring(s) of ISF extraction devices according to the present invention can provide a controlled flow of the extracted ISF sample. For example, retraction of the pressure ring(s) can stop ISF sample flow for a time period of 0.1 seconds to 60 minutes to allow a glucose concentration measurement to be conducted. Once the glucose concentration measurement is complete, one or more of the pressure rings can be redeployed to continue ISF extraction. In this manner, a semi-continuous ISF sample extraction can be accomplished.

Once apprised of the present disclosure, one skilled in the art will recognize that ISF extraction devices according to the present invention can be employed in a variety of systems including, but not limited to, systems for the extraction of a bodily fluid sample and monitoring of an analyte therein, as described above. For example, the ISF extraction devices can be employed in a sample module of such systems.

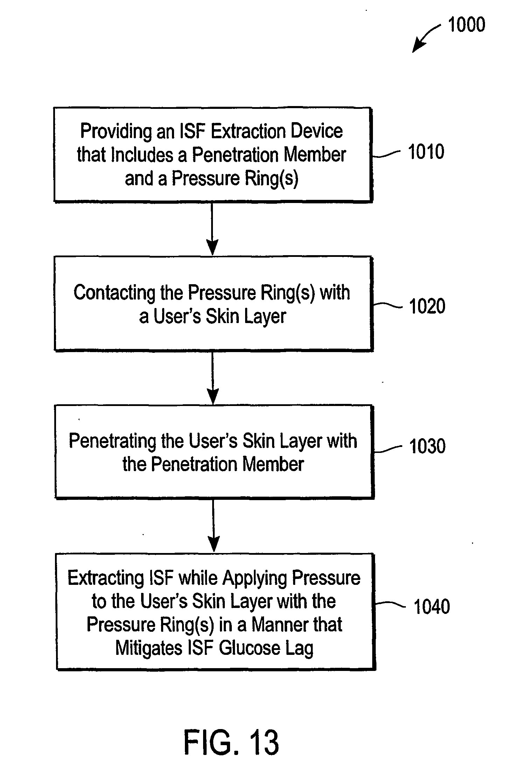

Referring to FIG. 13, a method 1000 for continuous collection of an ISF sample from a user's skin layer includes providing an ISF fluid extraction device, as set forth in step 1010. The ISF fluid extraction device that is provided includes a penetration member and at least one pressure ring (e.g., a single pressure ring or three concentric pressure rings). The penetration member and pressure ring(s) can be penetration members and pressure rings, as described above with respect to ISF extraction devices and systems according to the present invention.

Next, as set forth in step 1020, the pressure ring(s) is contacted with a user's skin layer in the vicinity of a target site (e.g., finger tip dermal tissue target site, a limb target site, an abdomen target site or other target site from which an ISF sample is to be extracted). The pressure ring can be contacted to the user's skin layer using any suitable techniques including, for example, those described above with respect to embodiments of systems and devices according to the present invention.

The target site of the user's skin layer is then penetrated by penetration member, as set forth in step 1030. Next, ISF is extracted from the user's skin layer by the penetration member while pressure is applied to the user's skin layer in an oscillating manner that mitigates an ISF lag of the extracted ISF, as set forth in step 1040. The various oscillating manners, by which pressure is applied, in methods according to the present invention have been described above with respect to FIGS. 1-12.

The following examples serve to illustrate beneficial aspects of various embodiments of devices and systems according to the present invention.

**Example 1: Impact of an oscillating pressure ring on blood perfusion in an area within the oscillating pressure ring**

Laser Doppler image perfusion data were collected at semi-regular intervals from a 0.25 square centimeter area approximately centered in the inside of a pressure ring attached to a subject's forearm. The pressure ring had an outside diameter 13.5 mm (0.53 inches) and a wall thickness of 0.76 mm (0.03 inches). Baseline data were collected prior to deploying the pressure ring against the subject's skin layer. The pressure ring was deployed against the skin layer for 10 minutes with a spring force of 0.07 N (0.5 lbs), retracted from the skin layer for 30 minutes, and then this cycle of deployment and retraction was repeated. The pressure ring was subsequently deployed against the skin layer for 5 hours, raised for 1 hour, and finally deployed against the skin for 10 minutes. The average perfusions in the 0.25 cm sq. measurement area are shown in the graph of FIG. 12.

As can be seen in the graph in FIG. 12, deployment of the pressure ring reduced blood perfusion in the area enclosed by the pressure ring (i.e., blood perfusion was reduced with the application of pressure), in comparison to the baseline blood perfusion. However, removing the pressure ring (i.e., releasing the pressure) not only reversed this effect, but actually increased perfusion beyond the baseline.

**Example 2: Impact of an oscillating pressure ring on ISF glucose lag**

A study was performed to determine the impact of blood flow on ISF glucose values during use of an oscillating pressure ring according to exemplary embodiments of the present invention. Twenty diabetic subjects underwent a procedure, in which baseline blood perfusion measurements were made on volar and dorsal portions of the subject's forearms. The subjects then participated in a test, in which finger blood samples, control ISF samples and treated ISF samples were collected at 15 minute intervals over a period of 3 to 6 hours. Control ISF samples were obtained from the subject's forearms without any skin layer manipulation and treated ISF samples were obtained by manipulating the subject's skin layer with an oscillating pressure ring. During the 3 to 6 hour testing period, blood glucose was influenced by ingestion of a microwave meal and diabetes medications including insulin and oral hypoglycemics such that most subjects experienced a rise and fall in blood glucose.

The treated ISF samples were created by applying approximately 1034 kPa (150 pounds per square inch) of pressure with a pressure ring with no sampling for 30 seconds, followed by a 5 minute waiting period to allow blood to perfuse into the sampling target site. Blood perfusion measurements were made with a Moor Laser Doppler Imager (Devon, UK) immediately prior to obtaining both control and treated ISF samples. Laser Doppler imaging was performed over a 2 square centimeter area centered on the ISF sampling target site.

ISF glucose measurements were made with a modified OneTouch® Ultra® glucose meter and test strip system.

A sample of about 1 μL of ISF was extracted from the dermis of the subject's skin layer by a needle and deposited automatically into a measurement zone of the test strip. An unmodified OneTouch® Ultra® glucose meter and strip system was used to determine whole blood glucose values from the finger.

Lag times in minutes and perfusion measurements are given in Table 1 for each subject.

**TABLE 1**

| Subject ID | control area mean blood perfusion units | treatment area mean blood perfusion units | treatment to control blood perfusion ratio | control ISF overall lag (min.) | treatment ISF overall lag (min.) | overall lag mitigation (min.) |
|---|---|---|---|---|---|---|
| 8 | 97.1 | 212.9 | 2.19 | 30 | 10 | 20 |
| 9 | 65.3 | 170.3 | 2.61 | 21 | 5 | 16 |
| 10 | 84.0 | 187.6 | 2.23 | 26 | 4 | 22 |
| 11 | 50.2 | 117.3 | 2.34 | 22 | -5 | 27 |
| 12 | 68.4 | 223.5 | 3.27 | 12 | -2 | 14 |
| 13 | 95.4 | 295.2 | 3.09 | 30 | 15 | 15 |
| 14 | 62.0 | 150.3 | 2.42 | 47 | 12 | 35 |
| 15 | 51.7 | 92.8 | 1.80 | 50 | 10 | 40 |
| 16 | 80.0 | 80.9 | 1.01 | 41 | 24 | 17 |
| 17 | 64.6 | 107.9 | 1.67 | 46 | 12 | 34 |
| 18 | 101.2 | 244.4 | 2.41 | 50 | 11 | 39 |
| 19 | 86.2 | 142.4 | 1.65 | 27 | 16 | 11 |
| 20 | 114.8 | 256.9 | 2.24 | 42 | 16 | 26 |
| 21 | 118.6 | 198.3 | 1.67 | 13 | 5 | 8 |
| 22 | 73.2 | 156.2 | 2.13 | 25 | 8 | 17 |
| 23 | 114.7 | 278.2 | 2.43 | 30 | 8 | 22 |
| 24 | 94.4 | 253.6 | 2.69 | 15 | 8 | 7 |
| 25 | 161.2 | 482.0 | 2.99 | 8 | -2 | 10 |
| 26 | 58.7 | 151.7 | 2.59 | 42 | 9 | 33 |
| 27 | 114.6 | 363.3 | 3.17 | 29 | 8 | 21 |
| 28 | 56.3 | 117.0 | 2.08 | 31 | 10 | 21 |
| **mean:** | **86.3** | **203.9** | **2.32** | **30.3** | **8.7** | **21.7** |
| SD: | 28.1 | 97.2 | 0.6 | 12.8 | 6.6 | 9.9 |

The data in Table 1 show that ISF glucose lag was mitigated an average of 21.7 minutes, i.e., from a mean of 30.3 minutes (12.8 SD) to a mean of 8.7 minutes (6.6 SD) by use of the oscillating pressure ring. This lag mitigation was accomplished by the application and release of pressure to the subject's skin layer in a manner that caused an elevation of local blood perfusion in the ISF sampling areas by an average of 2.3 times (0.6 SD) relative to control sampling areas.

**Example 3: Assessment of calibration methodology and its impact on accuracy of an ISF glucose sensor**

A study was performed to assess various calibration methodologies and their impact on system accuracy. A diabetic subject underwent a study, in which measurements of glucose were made from three sample types collected in parallel at fifteen minute intervals (i.e. measurement cycles) over a 5.5 hour period. During the study, a glucose excursion was induced through oral ingestion of a 75 g dextrose solution.

The three sample types collected for glucose measurement were finger blood samples, control ISF samples,

and treated ISF samples. Finger blood samples, which may also be referred to as finger capillary blood (FCB), were collected by standard finger lancing. Control ISF samples (CISF) were collected from the subject's arm without any skin layer manipulation and treated ISF samples (TISF) were collected from the subject's other arm with skin layer manipulation using an oscillating pressure ring. All sample collection times were recorded by computer time stamping, resulting in data pairs (i.e. measurement cycle number and a glucose concentration) for each of the sample types. The glucose concentration of FCB, which is abbreviated as $[G]_{FCB}$, was measured in duplicate by using two One Touch® Ultra blood glucose meters and test strips (LifeScan, Milpitas, CA). Reported values are the means of the two meter readings for each sample.

The collection of the two ISF sample types differed in methodology. CISF was collected from one of subject's arm in a way such that a different site on the dorsal forearm was sampled for each time interval. A sampling module is employed that includes a pressure ring, a small gauge needle, and an adapter for interfacing to a glucose test strip. Approximately one microliter of ISF was collected through a 30 gauge needle penetrating into the dermal layer to a skin depth of about 2 millimeters. Application of about 15 Newtons of force on the skin through a 5.5 mm diameter pressure ring facilitated collection of CISF (median collection time 3.0 sec), which was deposited in the measurement zone of a modified One Touch® Ultra glucose measurement strip. The inlet area of the strip was physically modified to interface with the adapter of the sampling module so that CISF could be directly deposited in the strip measurement zone.

TISF was collected on a sampling module which was slightly different than the one used for CISF. This sampling module was mounted on the subject's dorsal forearm. More specifically, the arm used for collecting TISF was the arm which was not used for collecting CISF. In contrast to the collection of CISF, TISF was collected from the same site for each time interval. This sampling module, which was adhered to the arm using a medical grade adhesive patch, included a 25 gauge needle designed for penetrating the skin to a depth of about 2 mm, and also had a pressure ring surrounding the needle, which was pushed towards the skin to collect TISF. The sampling module further included a reservoir for accumulating TISF. In this test, the reservoir was 0.5 µL glass capillary tubes (Drummond Scientific, Broomall, PA) in which a 320 nL volume is collected which matches the swept volume of the needle. Once the requisite volume of ISF was collected, the capillary tube was removed and TISF was transferred onto a different type of modified One Touch® Ultra glucose measurement strip. This second strip modification allowed for the direct capillary tube expression of TISF to the measurement zone which allowed a smaller volume to be measured than the strips used for CISF. In this second strip modification, only one working electrode was used (as opposed to using two working electrodes), and the area of the working and reference electrode were decreased to accommodate the relatively low sample size. It should be noted that pressure is applied only during the collection of the 320 nL sample which is typically about 85 seconds. After the requisite volume is collected, the pressure ring changes to the retracted state in which the needle continues to reside in the dermis. No additional pressure is applied for the balance of the 15 minute interval.

Table 2 shows the data collected for the three sample types collected from the diabetic subject over 22 measurement cycles. The results of FCB sample results are shown as a glucose concentration (i.e. $[G]_{FCB}$) in units of mg/dL. The results of CISF and TISF are shown as a current in units of nanoamps, which was respectively abbreviated as $i_{CISF}$ and $I_{TISF}$. To simplify the format of the data, $i_{CISF}$ and $i_{TISF}$ were normalized for differences in electrode area so that they can be directly comparable and employ the same calibration equation. In addition, $i_{CISF}$ and $i_{TISF}$ values were converted to a series of glucose concentrations using a previously calculated calibration equation. The glucose concentrations for CISF and TISF are shown in units of mg/dL and were respectively abbreviated as $[G]_{CISF}$ and $[G]_{TISF}$.

**Table 2.**

| Measurement cycle | $[G]_{FCB}$(mg/dL) | $i_{CISF}$ (nA) | $i_{TISF}$ (nA) | $[G]_{CISF}$ (mg/dL) | $[G]_{TISF}$ (mg/dL) |
|---|---|---|---|---|---|
| 1 | 107 | 241 | | 65 | |
| 2 | 104 | 436 | 361 | 124 | 101 |
| 3 | 110 | 428 | 401 | 121 | 113 |
| 4 | 200 | 422 | 644 | 119 | 186 |
| 5 | 311 | 505 | 1008 | 144 | 296 |
| 6 | 362 | 804 | 1171 | 234 | 345 |
| 7 | 369 | 908 | 1272 | 265 | 375 |
| 8 | 338 | 916 | 1182 | 268 | 348 |
| 9 | 354 | 916 | 1275 | 268 | 376 |
| 10 | 345 | 1011 | 1109 | 296 | 326 |

(continued)

| Measurement cycle | $[G]_{FCB}$(mg/dL) | $i_{CISF}$ (nA) | $i_{TISF}$ (nA) | $[G]_{CISF}$ (mg/dL) | $[G]_{TISF}$ (mg/dL) |
|---|---|---|---|---|---|
| 11 | 354 | 958 | 1387 | 281 | 410 |
| 12 | 348 | 1122 | 1229 | 330 | 362 |
| 13 | 334 | 1007 | 1229 | 295 | 362 |
| 14 | 310 | 1106 | 1096 | 325 | 322 |
| 15 | 291 | 1216 | 1126 | 358 | 331 |
| 16 | 268 | 1053 | 1012 | 309 | 297 |
| 17 | 251 | 1074 | 1025 | 315 | 301 |
| 18 | 238 | 995 | 905 | 292 | 265 |
| 19 | 222 | 997 | 740 | 292 | 215 |
| 20 | 211 | 974 | 812 | 285 | 237 |
| 21 | 195 | 845 | 743 | 247 | 216 |
| 22 | 175 | 793 | 708 | 231 | 205 |

For the glucose measurement of CISF and TISF, the respective modified measurement strips were both calibrated with an ISF surrogate which allows the actual glucose concentration to be determined in CISF and TISF. ISF surrogate is a fluid derived from plasma that is intended to mimic ISF. The use of ISF surrogate in the calibration process is due to the fact that relatively large volumes (i.e. about a milliliter) of ISF are difficult to collect. The calibration process requires relatively large fluid volumes because several calibrants (typically six) must be prepared. ISF surrogate was prepared using plasma diluted 1:2 (500 microliters + 500 microliters) with isotonic saline. Appropriate volumes of 1 molar glucose solution were spiked into ISF surrogate to prepare six calibrants having a glucose concentration of 2.5, 5, 10, 20, and 30 mM. For each calibrant glucose concentration, at least 5 replicates were performed and an average current value was calculated at 5 seconds. Using routine linear regression, a slope and intercept was calculated for use in a calibration equation which converts current into a glucose concentration. Because $i_{CISF}$ and $i_{TISF}$ were normalized for electrode area, a similar calibration equation was used for calculating $[G]_{CISF}$ and $[G]_{TISF}$ which is shown by eq. 1A and eq. 1B.

$$\text{eq. 1A} \qquad [G]_{CISF} = 0.3 \times i_{CISF} - 7.6 \text{ nA}$$

$$\text{eq. 1B} \qquad [G]_{TISF} = 0.3 \times i_{TISF} - 7.6 \text{ nA}$$

It should be noted that this type of calibration would most likely be performed by the manufacturer of the test strip.
A different type of calibration procedure will now be discussed for the purpose of accurately measuring glucose in ISF using a semi-continuous or continuous glucose sensor in systems according to the present invention. This type of calibration would most likely be performed by the user of the semi-continuous or continuous glucose sensor. For example, a calibration can be performed using only one glucose measurement with FCB and a single use glucose measurement strip such as a One Touch® Ultra glucose measurement strip. In such a situation, a simple proportion can be calculated for estimating $[G]_{CISF}$ using FCB which is abbreviated as $[G]_{CISF,FCB}$. As an arbitrary time interval, measurement cycle 6 was used for performing the one point calibration with FCB. It should be noted that measurement cycle 6 which represents a situation in which $[G]_{FCB}$ is rising with time and will be shown to be problematic calibration interval in the absence of lag mitigation, but nonetheless represents a possible time interval that a user may select. Using a simple proportion, the calibration equation can be represented by eq. 2.

$$\text{eq. 2} \qquad [G]_{CISF,FCB} = i_{CISF} \, x \, \frac{[G]_{FCB,6}}{i_{CISF,6}} = i_{CISF} \, x \, \frac{362}{804} = i_{CISF} \, x0.45$$

In eq. 2, $[G]_{FCB,6}$ represents the finger capillary blood glucose concentration at the sixth measurement cycle and $i_{CISF,6}$ represents the current measured for a CISF sample at the sixth measurement cycle. Because the glucose concentrations in ISF tend to lag behind the glucose concentrations in FCB, the use of a FCB calibration effectively predicts what the ISF glucose concentration will be in the future.

For simplicity purposes, the analysis of only a portion of Table 2 will be described in this example and following examples. Measurement cycles 5, 12 and 21 will be further analyzed and respectively referred to hereinafter as "rising", "stable", and "falling". Table 3 shows a comparison of $[G]_{CISF,FCB}$ and $[G]_{CISF}$ for the three previously mentioned measurement cycles. The data indicates that there is a relatively large absolute error between an ISF glucose sensor measuring CISF using a one point FCB calibration and a factory calibration using 6 ISF surrogate calibrants.

Table 3. Comparison of one point FCB calibration vs. factory calibration using a CISF sample.

| Measurement cycle | $[G]_{CISF,FCB}$ (mg/dL) | $[G]_{CISF}$ (mg/dL) | Absolute Error |
|---|---|---|---|
| Rising | 227 | 144 | 83 |
| Stable | 505 | 330 | 175 |
| Falling | 380 | 247 | 134 |

In addition to CISF, TISF can also be analyzed for its glucose concentration using a one point FCB calibration. For such a case, eq. 3 can be derived for predicting the glucose concentration of TISF using FCB, which is abbreviated as $[G]_{TISF,FCB}$.

$$\text{eq. 3} \qquad [G]_{TISF,FCB} = i_{TISF} \, x \, \frac{[G]_{FCB,6}}{i_{TISF,6}} = i_{TISF} \, x \, \frac{362}{1171} = i_{TISF} \, x0.309$$

Similar to eq. 2, eq. 3 also used measurement cycle 6 for performing the calibration with FCB. Table 4 shows a comparison of $[G]_{TISF,FCB}$ and $[G]_{CISF}$ for the three measurement cycles. The absolute error (83 to 175 mg/dL) between an ISF glucose sensor measuring TISF using a one point FCB calibration and a factory calibration using 6 ISF surrogate calibrants is smaller than the overall absolute error (14-18 mg/dL) shown in Table 3. Therefore, Tables 3 and 4 demonstrate the utility of ISF glucose lag mitigation when using FCB to calibrate an ISF glucose sensor for the future prediction of ISF glucose concentrations.

Table 4. Comparison of one point FCB calibration vs. factory calibration using a TISF sample.

| Measurement cycle | $[G]_{TISF,FCB}$ (mg/dL) | $[G]_{TISF}$ (mg/dL) | Absolute Error |
|---|---|---|---|
| Rising | 311 | 296 | 16 |
| Stable | 380 | 362 | 18 |
| Falling | 230 | 216 | 14 |

ISF glucose concentration measurements can be used to predict the glucose concentration in FCB. In general, physicians may prefer to use the glucose concentration in FCB as the basis for determining the appropriate therapy for helping control the disease state because this is historically what has been done. However, a large proportion of the continuous and minimally invasive glucose sensors that have been commercialized or are in the process of being commercialized use mainly ISF and not blood. Therefore, there is a need for estimating the glucose concentration in capillary blood using a continuous or semi-continuous ISF glucose sensor.

Table 5 shows a comparison of $[G]_{CISF,FCB}$ and $[G]_{FCB}$ for the three measurement cycles. The data shows that the absolute error is relatively large when trying to estimate the glucose concentration in FCB using a CISF measurement calibrated with FCB.

Table 5. Accuracy assessment of a CISF measurement using one point FCB for estimating the glucose concentration in FCB

| Measurement cycle | [G]$_{CISF,FCB}$ (mg/dL) | [G]$_{FCB}$ (mg/dL) | Absolute Error |
|---|---|---|---|
| Rising | 227 | 311 | 84 |
| Stable | 505 | 348 | 157 |
| Falling | 380 | 195 | 185 |

Table 6 shows a comparison of [G]$_{CISF,TISF}$ and [G]$_{FCB}$ for the three measurement cycles. [G]$_{CISF,TISF}$ represents the glucose concentration in a CISF sample that was calibrated using a TISF sample and a FCB sample. An eq. 4 was developed to calculate [G]$_{CISF,TISF}$.

$$\text{eq. 4} \qquad [G]_{CISF,TISF} = i_{CISF}\,x\,\frac{[G]_{FCB,6}}{i_{TISF,6}} = i_{TISF}\,x\,\frac{362}{1171} = i_{CISF}\,x\,0.309$$

Table 6. Accuracy assessment of a CISF measurement using a TISF sample and a FCB sample for estimating the glucose concentration in FCB

| Measurement cycle | [G]$_{CISF,TISF}$ (mg/dL) | [G]$_{FCB}$ (mg/dL) | Absolute Error |
|---|---|---|---|
| Rising | 156 | 311 | 155 |
| Stable | 347 | 348 | 1 |
| Falling | 261 | 195 | 66 |

A comparison of Table 5 and 6 show that the measurement of glucose in CISF gives a better estimate of capillary blood glucose concentration when the ISF sensor is calibrated with TISF and FCB. For the case using a TISF sample and a FCB sample, the absolute error is lower for the "stable" and "falling" measurement cycles in Table 5 when compared to the case using only a FCB sample in Table 6. The absolute error is higher for the "rising" measurement cycle in Table 6. However, the overall average error is smaller for the case in Table 6 which employs some lag mitigation (74 mg/dL in Table 6 vs. 142 mg/dL in Table 5). Therefore, even though CISF is collected and tested without lag mitigation, there is still an improvement in being able to estimate capillary glucose concentrations if the ISF sensor is calibrated with TISF and FCB.

[0163]    [00171] Table 7 shows a comparison of [G]$_{TISF}$ and [G]$_{FCB}$ for the three measurement cycles. The data shows that the absolute error is smaller for estimating the glucose concentration in FCB using a TISF sample (0 to 35 mg/dL, see Table 7) instead of a CISF sample (84 to 185 mg/dL, see Table 5), both of which were calibrated using FCB. Therefore, the use of lag mitigation is clearly superior in accuracy when estimating capillary blood glucose concentrations using an ISF glucose sensor.

Table 7. Accuracy assessment of a TISF measurement using one point. FCB calibration for estimating the glucose concentration in FCB

| Measurement cycle | [G]$_{TISF,FBC}$ (mg/dL) | [G]$_{FCB}$ (mg/dL) | Absolute Error |
|---|---|---|---|
| Rising | 311 | 311 | 0 |
| Stable | 380 | 348 | 32 |
| Falling | 230 | 195 | 35 |

Although disposable test strips are described in this example to measure ISF glucose, the calibration concepts discussed herein also apply to any sensor which measures ISF glucose especially semi-continuous and continuous glucose sensors. The previously described calibration methodologies show that use of lag mitigation prior to calibration improves accuracy for estimating either CISF, TISF, or capillary glucose concentrations. Therefore, once apprised of the present disclosure, one skilled in the art will recognize that the calibration algorithms (equations) described in this example can be employed in systems according to embodiments of the present invention. For example, the calibration algorithms can be employed in sampling or analysis modules to calculate capillary blood glucose concentrations based on ISF measurement data.

**Example 4: ISF Glucose Lag Mitigation Methodology by Pressure Ring Cycling**

Twenty-two diabetic subjects (12 male, 10 female; nine Type 1, 13 Type 2; median age 53.5 years; median Body Mass Index (BMI) 25.4; median time since onset: 18.0 years) participated in an ethics committee approved test in which measurements of glucose were made from three samples collected at fifteen minute intervals (a measurement cycle) over a five to six hour period.

During the test, a glucose excursion was induced through oral ingestion of either a 75 g dextrose solution (by 12 subjects, deemed the "75g load subjects") or normal eating habit (by 10 other subjects, deemed the "NEH subjects"). Subjects managed the ingestion with their prescribed insulin injections or oral medications.

The three samples for glucose measurement were finger capillary blood sampled by standard finger capillary blood lancing, and two ISF samples (control and test ISF samples as described below), one from each arm of each subject. All sample collection times were recorded by computer time stamping, resulting in (time, glucose) data pairs for each of the samples at each of the measurement intervals. Finger capillary blood glucose was measured in duplicate by two One Touch® Ultra blood glucose meters (available from LifeScan, Milpitas, CA). The glucose values reported herein are the means of the two meter readings for each sample.

The collection of the ISF samples from each arm differed in methodology. On one arm (randomly selected), designated the control ISF arm, each discrete sample of ISF was collected from a different sampling site on the dorsal forearm. Approximately one microliter of ISF was collected through a small gauge needle penetrating into the dermal layer to a skin depth of ~2 mm. Application of ~15 N of force on the skin through a 5.5 mm diameter pressure ring facilitated collection of the ISF sample (median collection time 3.0 sec, N=553), which was subsequently deposited in the measurement zone of a modified One Touch® Ultra glucose measurement strip for glucose measurement. The strips were modified to interface with an adapter for the ISF sampling system so that the ISF could be directly sampled and deposited in the strip measurement zone.

On the other arm, designated the test ISF arm, a prototype continuous ISF collection device was mounted on the dorsal forearm. This device, which was adhered to the arm using a medical grade adhesive patch, consisted of a small gauge needle penetrating the skin to a depth of about 2 mm, and also a pressure ring surrounding the needle, which was pushed into the skin to collect a sample of ISF. In this test, ISF samples of 320 nL, equivalent to the swept volume of the needle, were collected into 0.5 $\mu$L glass capillary tubes (commercially available from Drummond Scientific, Broomall, PA).

Once the requisite volume of ISF was collected, the capillary tube was removed and the ISF expressed onto a modified One Touch® Ultra glucose measurement strip to measure glucose. This second strip modification allowed for the direct capillary tube expression of the sample in the measurement zone, allowing a smaller volume to be measured than is usual for these strips. For both ISF glucose measurements, the modified measurement strips were prospectively calibrated with an ISF surrogate so that ISF glucose was directly determined for both the control ISF and test ISF samples.

For the collection of the test ISF samples, pressure was applied only during the collection of the 320 nL sample (median collection time 85 sec, N=530). After the requisite volume was collected, the application of pressure to the ring surrounding the needle was stopped, although the needle continued to reside in the dermis. No more pressure was applied for the balance of each 15-minute cycle interval.

For the comparison of the ISF and blood glucose values on a time basis, it is desired to match the times at which each sample is obtained from the body with its glucose value. For the test ISF samples, this means that a one-cycle time axis shift was performed to account for the fact that the 320 nL ISF sample actually collected during a particular cycle had been residing in the needle (dead volume 320 nL) since the previous collection cycle. In this way, an accurate measure of physiological lag can be made relative to finger blood samples collected at the same relative time.

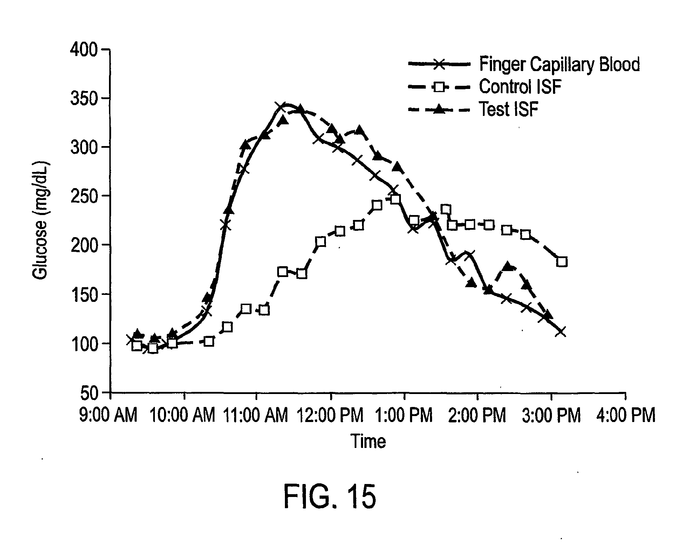

An exemplary time course plot obtained for one subject is shown in FIG. 15. This shows the results for the glucose measurements in the three samples plotted vs. time. With the one cycle time shift for the test ISF, the time axis accurately represents the time at which each of the three samples was extracted from the body. The time shift accounts for the fact that, in the case of the test ISF, the sample is extracted from the body, but still resident in the 320 nL bore of the cannula, waiting to be pushed into the capillary tube for the next time point measurement. Therefore, the plot accurately reflects the physiological glucose lag between the ISF and blood samples.

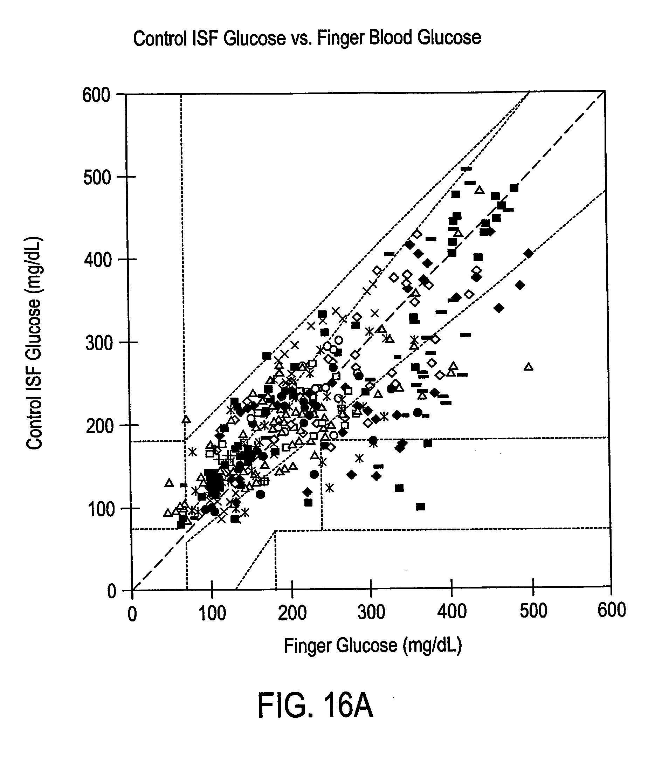

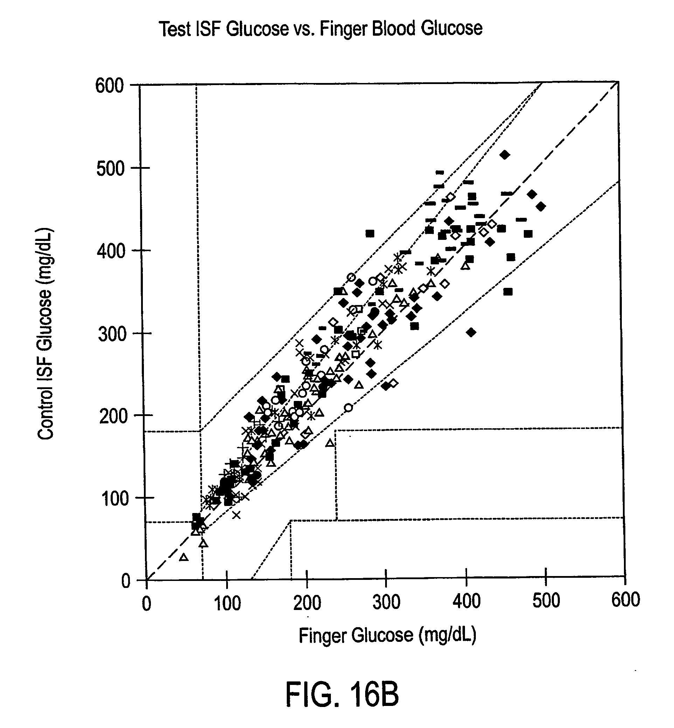

A comparison of all of the data collected for the 22 subjects is shown in FIGs. 16A and 16B, which shows method comparison plots superimposed on a Clarke Error Grid. Clarke Error Grid statistics, regression statistics (slope, intercept and correlation coefficient, R), standard error between the blood and ISF values (Sy.x), average percent bias and mean percent absolute error (MPAE) between the reference finger blood glucose values and ISF glucose values are shown in Table 8. By all measures the test ISF provides a better estimate of blood glucose than the control ISF.

TABLE 8

| statistic | control ISF | test ISF |
|---|---|---|
| % in A | 53.9% | 72.3% |
| % in B | 39.6% | 26.3% |
| % in C | 0.2% | 0.9% |
| % in D | 6.3% | 0.0% |
| % in E | 0.0% | 0.0% |
| slope: | 0.69 | 0.99 |
| intercept: | 64.7 | 22.2 |
| Sy.x | 52.5 | 34.1 |
| R | 0.81 | 0.95 |
| avg.bias (%): | 4.9 | 10.0 |
| MPAE: | 22.3 | 14.6 |

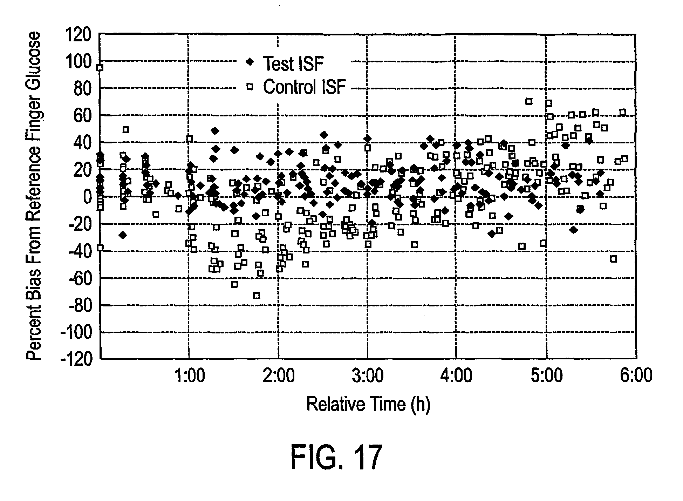

It is noted that there may be a significant systematic bias in the test ISF measurements. FIG. 17 shows a plot of ISF measurement bias relative to the reference finger blood values for both of the ISF measurements, plotted vs. time of sample collection during the testing, where zero time is the start of each test. The plot shows the data for the twelve 75 g load subjects, since glycemic range and trending were greater for these subjects than the NEH subjects, and so serve best to illustrate the point. The roughly sinusoidal bias pattern for the control ISF measurements mirrors the time course plots, i.e., generally negative bias during the period of rising blood glucose, turning to generally positive bias towards the end of the test when glucose is falling. The test ISF, however, has a generally flat bias response vs. test time, with an average bias of 10.7% (10.0% overall, including all subjects, see Table 8). This flat bias response potentially indicates a simple calibration offset, which can easily be corrected by subtracting 10% from all test ISF values.

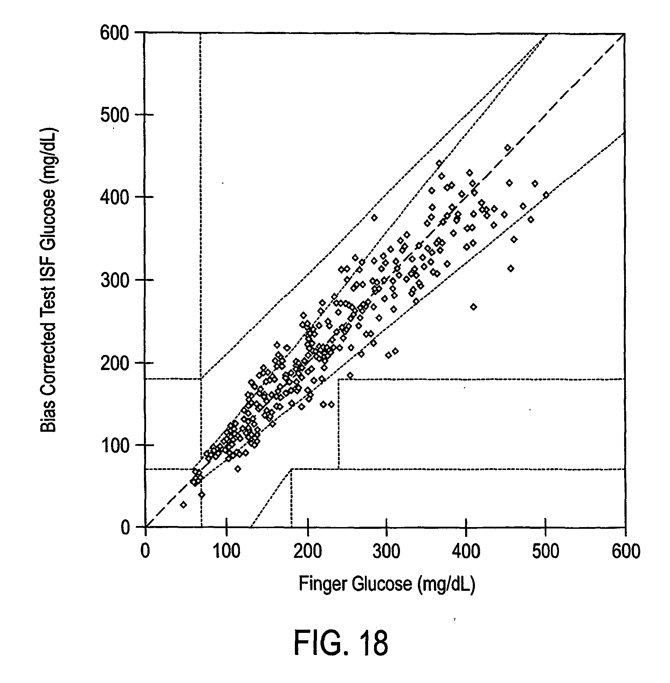

FIG. 18 shows the regression plot of the test ISF glucose vs. reference finger blood glucose when this bias correction is performed, and Table 9 shows the Clarke Error Grid, regression, and error statistics when this mean centering bias correction is applied to both test (10% bias correction from Table 8) and control ISF (4.9% bias correction from Table 8) measurements. The bias correction for the control ISF has little effect on the overall accuracy. However, there is a considerable improvement on the overall accuracy for the test ISF when the bias correction is applied. This indicates that a major component of error for the test ISF measurements is likely a simple calibration error, which can be solved through more rigorous calibration methodology.

TABLE 9

| statistic | bias corr. control ISF | bias corr. test ISF |
|---|---|---|
| % in A | 54.3% | 85.8% |
| %inB | 38.7% | 14.2% |
| % in C | 0.2% | 0.0% |
| % in D | 6.7% | 0.0% |
| % in E | 0.0% | 0.0% |
| slope: | 0.65 | 0.89 |
| intercept: | 64.0 | 20.0 |
| Sy.x | 53.4 | 30.7 |
| R | 0.79 | 0.95 |
| avg.bias (%): | 1.2 | -1.0 |
| MPAE: | 22.2 | 10.9 |

The fact that the control ISF measurements were little affected (as is evident from a comparison of Table 8 and Table 9 control ISF results) indicates that any calibration error is a minor component of error for these measurements. These results show the potential for improvement in ISF glucose measurements relative to finger blood glucose measurements when a treatment such as the slow pressure ring modulation is applied to the ISF sampling area.

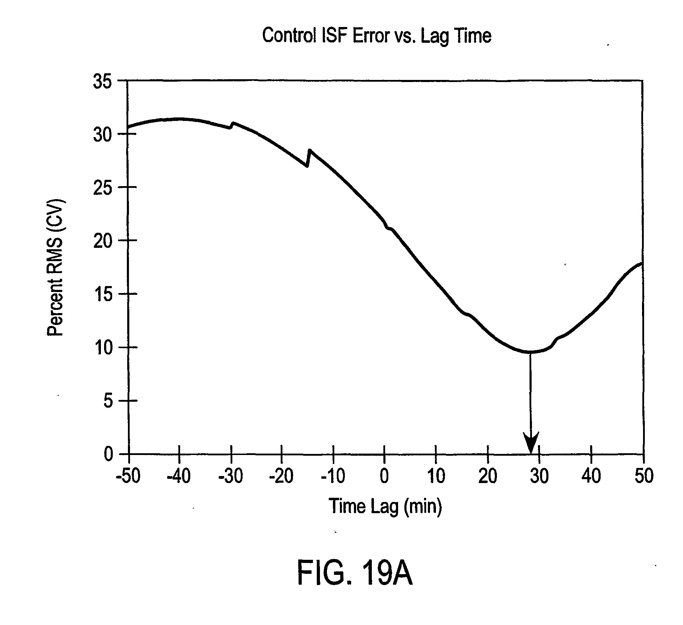

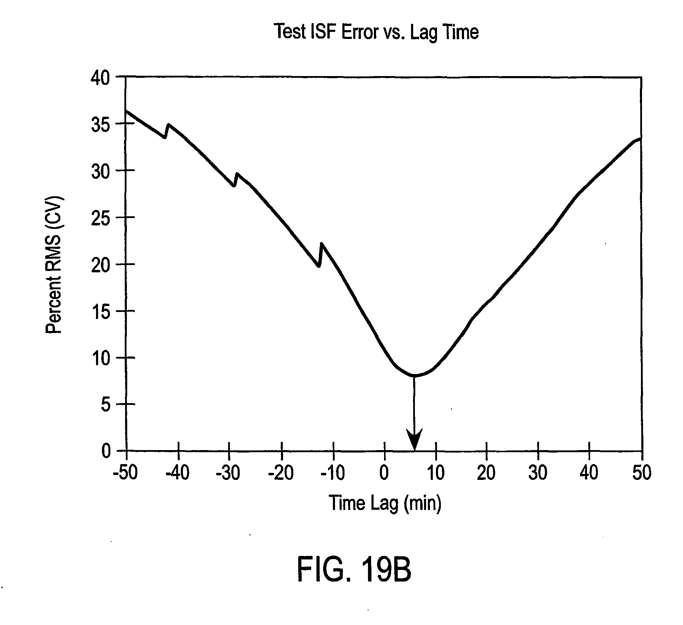

The average glucose lag time between each of the ISF samples and the reference finger blood samples was calculated for each subject as a way of determining the amount of lag mitigation achieved by the continuous ISF extraction device when compared to the lag of the discretely sampled control ISF samples. The lag between ISF and blood glucose was measured by finding the minimum error between these measurements when the time axis for the ISF measurements is slid relative to the time axis of the blood measurements. The distance (in time) that the time axis is slid to achieve the minimum error is the average measured lag for a particular subject. This method was previously used to calculate an average control ISF lag time of 25 minutes across 57 diabetic subjects. The method was modified for individual subject calculation rather than a composite data set calculation. For example, FIGs. 19A and 19B show the error vs. time plots used to determine the average control and test ISF lag times for one subject in the current test.

Table 10 shows a summary of the individual subject calculated average lag times for each of the two ISF samples relative to finger blood glucose. Only 15 of the 22 subjects are represented here. For the other seven subjects (one of the 12 in the 75 g load group, and six of the 10 NEH subjects), either not enough data were available for the calculation or they did not display enough change in glycemic range in order to make a meaningful lag determination. As the table shows, there is a remarkable reduction in lag time for the test ISF samples relative to the control ISF samples for every subject. On average, a lag reduction of 35.8 minutes is achieved, cutting the average lag from 38.3 to 2.5 minutes, or a 95% reduction of the physiological lag.

TABLE 10

| Subject | Test Type | Control ISF lag (minutes) | Test ISF Lag (minutes) | Lag Difference (minutes) | % Lag mitigation |
|---|---|---|---|---|---|
| 1 | 75g load | 38 | -3 | 41 | 108% |
| 2 | 75g load | 42 | -1 | 43 | 102% |
| 3 | 75g load | 40 | 15 | 25 | 63% |
| 4 | 75g load | 28 | -3 | 31 | 111% |
| 5 | 75g load | 28 | 6 | 22 | 79% |
| 6 | 75g load | 39 | 3 | 36 | 92% |
| 7 | 75g load | 60 | 1 | 59 | 98% |
| 8 | 75g load | 50 | 9 | 41 | 82% |
| 9 | 75g load | 42 | 8 | 34 | 81% |
| 10 | 75g load | 40 | -8 | 48 | 120% |
| 11 | 75g load | 60 | 10 | 50 | 83% |
| 12 | NEH | 28 | 1 | 27 | 96% |
| 13 | NEH | 27 | 2 | 25 | 93% |
| 14 | NEH | 27 | -8 | 35 | 130% |
| 15 | NEH | 25 | 5 | 20 | 80% |
| | All subjects | 38.3 | 2.5 | 35.8 | 95% |
| | combined | 11.5 | 6.6 | 11.3 | 18% |
| | 75g load | 42.5 | 3.4 | 39.1 | 93% |
| | subjects | 1.3 | 5.6 | 6.2 | 21% |

(continued)

| Subject | Test Type | Control ISF lag (minutes) | Test ISF Lag (minutes) | Lag Difference (minutes) | % Lag mitigation |
|---|---|---|---|---|---|
| | NEH | 26.8 | 0.0 | 26.8 | 100% |
| | subjects | 1.3 | 5.6 | 6.2 | 21% |

Interestingly, the natural biases between ISF and blood glucose appears to be significantly reduced by a method that includes blood perfusion elevation, such as the modulated pressure ring application methodology applied in the test described here. It is, therefore, hypothesized that the modulated pressure application in this test acts to increase blood perfusion around the ISF sampling site, acting to significantly mitigate the physiological lag (i.e., ISF glucose lag).

While preferred embodiments of the present invention have been shown and described herein, it will be obvious to those skilled in the art that such embodiments are provided by way of example only. Numerous variations, changes, and substitutions will now occur to those skilled in the art without departing from the invention.

A system for monitoring an analyte in Interstitial Fluid (ISF) of a user, the system comprising: a cartridge including an analysis module for measuring an analyte in the ISF of the user; and a local controller module in electronic communication with the cartridge, the local controller configured to receive measurement data from the analysis module and store the data, wherein the analysis module includes an analyte sensor configured to be at least partially implanted in a target site of the user, and wherein the analysis module includes at least one pressure ring adapted for applying pressure to the body in the vicinity of the target site, and wherein the analysis module is configured such that the pressure ring is capable of applying the pressure in an oscillating manner whereby an ISF glucose lag is mitigated. The analysis module of the aforementioned system herein employs a lag mitigating chemical to further mitigate the ISF glucose lag. The analysis module of the aforementioned system herein employs ultrasound to further mitigate the ISF glucose lag. The analysis module of the aforementioned system herein employs heat to further mitigate the ISF glucose lag. The analysis module of the aforementioned system herein employs vacuum to further mitigate the ISF glucose lag. The analysis module of the aforementioned system herein employs an electropotential to further mitigate the ISF glucose lag. The analysis module of the aforementioned system herein employs non-oscillatory mechanical manipulation of the body to further mitigate the ISF glucose lag.

A system for extracting a bodily fluid sample and monitoring glucose therein, the system comprising: a disposable cartridge including: a sampling module for extracting a bodily fluid sample from a body; and an analysis module for measuring glucose in the bodily fluid sample; and a local controller module in electronic communication with the disposable cartridge, the local controller configured to receive measurement data from the analysis module and store the data, wherein at least one of the analysis module and the local controller module employs a calibration algorithm that depends on a glucose concentration measured from capillary blood and measurement data from the analysis module. In the aforementioned system in this paragraph the bodily fluid sample is an ISF sample and the measurement data from the analysis module is obtained with ISF glucose lag mitigation. The sampling module of the aforementioned system in this paragraph includes at least one pressure ring. The sampling module of the aforementioned system in this paragraph is configured such that the pressure ring is capable of applying the pressure in an oscillating manner whereby an ISF glucose lag is mitigated. The sampling module of the aforementioned system in this paragraph includes a penetration member, at least one pressure ring and the pressure ring is capable of applying the pressure in an oscillating manner whereby an ISF glucose lag is mitigated.

A system for monitoring an analyte in a bodily fluid of a user, the system comprising: a disposable cartridge including: an analysis module for measuring an analyte in the bodily fluid sample; and a local controller module in electronic communication with the disposable cartridge, the local controller configured to receive measurement data from the analysis module and store the data, wherein at least one of the analysis module and the local controller module employs a calibration algorithm that depends on a glucose concentration measured from capillary blood and measurement data from the analysis module. In the aforementioned system the bodily fluid sample is an ISF sample and the measurement data from the analysis module is obtained with ISF glucose lag mitigation. The sampling module of the aforementioned system in this paragraph includes at least one pressure ring. The sampling module of the aforementioned system in this paragraph is configured such that the pressure ring is capable of applying the pressure in an oscillating manner whereby an ISF glucose lag is mitigated. The sampling module of the aforementioned system in this paragraph includes a penetration member, at least one pressure ring and the pressure ring is capable of applying the pressure in an oscillating manner whereby an ISF glucose lag is mitigated.

A system for extracting a bodily fluid sample and monitoring an analyte therein, the system comprising: a disposable cartridge including: a sampling module for extracting a bodily fluid sample from a body; and an analysis module for measuring an analyte in the bodily fluid sample; and a local controller module in electronic communication with the disposable cartridge, the local controller configured to receive measurement data from the analysis module and store the data, wherein the sampling module employs a microdialysis-based sample extraction technique. The sampling module of the aforementioned system in this paragraph is configured to extract an interstitial fluid (ISF) sample and to measure glucose in the ISF sample and wherein the sampling module further includes means for mitigating ISF glucose lag. The means for mitigating ISF glucose lag of the aforementioned system in this paragraph employs a lag mitigating chemical. The means for mitigating ISF glucose lag of the aforementioned system in this paragraph employs ultrasound to mitigate ISF glucose lag. The means for mitigating ISF glucose lag of the aforementioned system in this paragraph employs heat to mitigate ISF glucose lag. The means for mitigating ISF glucose lag of the aforementioned system in this paragraph employs vacuum to mitigate ISF glucose lag. The means for mitigating ISF glucose lag of the aforementioned system in this paragraph employs an electropotential to mitigate ISF glucose lag. The means for mitigating ISF glucose lag of the aforementioned system in this paragraph employs mechanical manipulation of the body to mitigate ISF glucose lag. The means for mitigating ISF glucose lag of the aforementioned system in this paragraph employs a combination of at least two of a lag mitigating chemical, ultrasound, heat, vacuum, an electropotential, and mechanical manipulation of the body to mitigate ISF glucose lag.

A system for extracting a bodily fluid sample and monitoring an analyte therein, the system comprising: a disposable cartridge including: a sampling module for extracting a bodily fluid sample from a body; and an analysis module for measuring an analyte in the bodily fluid sample; and a local controller module in electronic communication with the disposable cartridge, the local controller configured to receive measurement data from the analysis module and store the data, wherein the sampling module employs an ultrafiltration-based sample extraction technique. The sampling module of the aforementioned system in this paragraph is configured to extract an interstitial fluid (ISF) sample and to measure glucose in the ISF sample and wherein the sampling module further includes means for mitigating ISF glucose lag. The means for mitigating ISF glucose lag of the aforementioned system in this paragraph employs an ISF glucose lag mitigating chemical. The means for mitigating ISF glucose lag of the aforementioned system in this paragraph employs ultrasound to mitigate ISF glucose lag. The means for mitigating ISF glucose lag of the aforementioned system in this paragraph employs heat to mitigate ISF glucose lag. The means for mitigating ISF glucose lag of the aforementioned system in this paragraph employs vacuum to mitigate ISF glucose lag. The means for mitigating ISF glucose lag of the aforementioned system in this paragraph employs an electropotential to mitigate ISF glucose lag. The means for mitigating ISF glucose lag of the aforementioned system in this paragraph employs mechanical manipulation of the body to mitigate ISF glucose lag. The means for mitigating ISF glucose lag of the aforementioned system in this paragraph employs a combination of at least two of a lag mitigating chemical, ultrasound, heat, vacuum, an electropotential, and mechanical manipulation of the body to mitigate ISF glucose lag.

A system for extracting a bodily fluid sample and monitoring an analyte therein, the system comprising: a disposable cartridge including: a sampling module for extracting a bodily fluid sample from a body; and an analysis module for measuring an analyte in the bodily fluid sample; and a local controller module in electronic communication with the disposable cartridge, the local controller configured to receive measurement data from the analysis module and store the data, wherein the sampling module employs a laser-based sample extraction technique. The sampling module of the aforementioned system in this paragraph is configured to extract an interstitial fluid (ISF) sample and to measure glucose in the ISF sample and wherein the sampling module further includes means for mitigating ISF glucose lag. The means for mitigating ISF glucose lag of the aforementioned system in this paragraph employs a lag mitigating chemical. The means for mitigating ISF glucose lag of the aforementioned system in this paragraph employs ultrasound to mitigate ISF glucose lag. The means for mitigating ISF glucose lag of the aforementioned system in this paragraph employs heat to mitigate ISF glucose lag. The means for mitigating ISF glucose lag of the aforementioned system in this paragraph employs vacuum to mitigate ISF glucose lag. The means for mitigating ISF glucose lag of the aforementioned system in this paragraph employs an electropotential to mitigate ISF glucose lag. The means for mitigating ISF glucose lag of the aforementioned system in this paragraph employs mechanical manipulation of the body to mitigate ISF glucose lag. The means for mitigating ISF glucose lag of the aforementioned system in this paragraph employs a combination of at least two of a lag mitigating chemical, ultrasound, heat, vacuum, an electropotential and mechanical manipulation of the body to mitigate ISF glucose lag.

A system for extracting a bodily fluid sample and monitoring an analyte therein, the system comprising: a disposable cartridge including: a sampling module for extracting a bodily fluid sample from a body; and an analysis module for measuring an analyte in the bodily fluid sample; and a local controller module in electronic communication with the disposable cartridge, the local controller configured to receive measurement data from the analysis module and store the data, wherein the sampling module employs a reverse iontophoresis-based sample extraction technique. The sampling module of the aforementioned system in this paragraph is configured to extract an interstitial fluid (ISF) sample and to measure glucose in the ISF sample and wherein the sampling module further includes means for mitigating ISF glucose lag. The means for mitigating ISF glucose lag of the aforementioned system in this paragraph employs a lag mitigating chemical. The means for mitigating ISF glucose lag of the aforementioned system in this paragraph employs ultrasound to mitigate ISF glucose lag. The means for mitigating ISF glucose lag of the aforementioned system in this paragraph employs heat to mitigate ISF glucose lag. The means for mitigating ISF glucose lag of the aforementioned system in this paragraph employs vacuum to mitigate ISF glucose lag. The means for mitigating ISF glucose lag of the aforementioned system in this paragraph employs an electropotential to mitigate ISF glucose lag. The means for mitigating ISF glucose lag of the aforementioned system in this paragraph employs mechanical manipulation of the body to mitigate ISF glucose. The means for mitigating ISF glucose lag of the aforementioned system in this paragraph employs a combination of at least two of a lag mitigating chemical, ultrasound, heat, vacuum, an electropotential, and mechanical manipulation of the body to mitigate ISF glucose lag.

A system for extracting a bodily fluid sample and monitoring an analyte therein, the system comprising: a disposable cartridge including: a sampling module for extracting a bodily fluid sample from a body; and an analysis module for measuring an analyte in the bodily fluid sample; and a local controller module in electronic communication with the disposable cartridge, the local controller configured to receive measurement data from the analysis module and store the data, wherein the sampling module employs an electroporation-based sample extraction technique. The sampling module of the aforementioned system in this paragraph is configured to extract an interstitial fluid (ISF) sample and to measure glucose in the ISF sample and wherein the sampling module further includes means for mitigating ISF glucose lag. The means for mitigating ISF glucose lag of the aforementioned system in this paragraph employs a lag mitigating chemical. The means for mitigating ISF glucose lag of the aforementioned system in this paragraph employs ultrasound to mitigate ISF glucose lag. The means for mitigating ISF glucose lag of the aforementioned system in this paragraph employs heat to mitigate ISF glucose lag. The means for mitigating ISF glucose lag of the aforementioned system in this paragraph employs vacuum to mitigate lag. The means for mitigating ISF glucose lag of the aforementioned system in this paragraph employs an electropotential to mitigate lag. The means for mitigating ISF glucose lag of the aforementioned system in this paragraph employs mechanical manipulation of the body to mitigate ISF glucose lag. The means for mitigating ISF glucose lag of the aforementioned system in this paragraph employs a combination of at least two of a lag mitigating chemical, ultrasound, heat, vacuum, an electropotential, and mechanical manipulation of the body to mitigate ISF glucose lag.

A system for extracting a bodily fluid sample and monitoring an analyte therein, the system comprising: a disposable cartridge including: a sampling module for extracting a bodily fluid sample from a body; and an analysis module for measuring an analyte in the bodily fluid sample; and a local controller module in electronic communication with the disposable cartridge, the local controller configured to receive measurement data from the analysis module and store the data, wherein the sampling module employs an ultrasound-based sample extraction technique. The sampling module of the aforementioned system in this paragraph is configured to extract an interstitial fluid (ISF) sample and to measure glucose in the ISF sample and wherein the sampling module further includes means for mitigating ISF glucose lag. The means for mitigating ISF glucose lag of the aforementioned system in this paragraph employs a lag mitigating chemical. The means for mitigating ISF glucose lag of the aforementioned system in this paragraph employs ultrasound to mitigate ISF glucose lag. The means for mitigating ISF glucose lag of the aforementioned system in this paragraph employs heat to mitigate ISF glucose lag. The means for mitigating ISF glucose lag of the aforementioned system in this paragraph employs vacuum to mitigate ISF glucose lag. The means for mitigating ISF glucose lag of the aforementioned system in this paragraph employs an electropotential to mitigate ISF glucose lag. The means for mitigating ISF glucose lag of the aforementioned system in this paragraph employs mechanical manipulation of the body to mitigate ISF glucose lag. The means for mitigating ISF glucose lag of the aforementioned system in this paragraph employs a combination of at least two of a lag mitigating chemical, ultrasound, heat, vacuum, an electropotential, and mechanical manipulation of the body to mitigate ISF glucose lag.

A system for monitoring an analyte in a bodily fluid of a user, the system comprising: a disposable cartridge including an analysis module for measuring an analyte in the bodily fluid sample; and a local controller module in electronic communication with the disposable cartridge, the local controller configured to receive measurement data from the analysis module and store the data, wherein the analysis module includes an analyte sensor configured to be at least partially implanted in the user. The analyte sensor of the aforementioned system in this paragraph is an ISF glucose analyte sensor and wherein the analysis module further includes means for mitigating glucose lag. The means for mitigating ISF glucose lag of the aforementioned system in this paragraph is at least one pressure ring adapted for applying pressure to the user while the analyte sensor is at least partially implanted in the user.

It should be understood that various alternatives to the embodiments of the invention described herein may be employed in practicing the invention. It is intended that the following claims define the scope of the invention and that methods and structures within the scope of these claims and their equivalents be covered thereby.

**Claims**

1. A system (10) for extracting an Interstitial Fluid (ISF) sample and monitoring an analyte therein, the system comprising:

a cartridge (12) including:

a sampling module (18) adapted to extract an ISF sample from a target site (TS) of a body (B), and
an analysis module (20) adapted to measure an analyte in the ISF sample; and

a local controller module 14 in electronic communication with the cartridge (12), the local controller being configured to receive measurement data from the analysis module (20) and store the data,

wherein:

the sampling module (18) includes a penetration member and at least one pressure ring (28 ; 904 ; 954 A-C) adapted for applying pressure to the body (B) in the vicinity of the target site (TS), and
the sampling module (18) is configured such that the at least one pressure ring (28; 904; 954 A-C) is capable of applying the pressure in an oscillating manner whereby an ISF glucose lag of the ISF sample extracted by the sampling module (18) is mitigated,

**characterised in that** the penetration member (902) is fixed with respect to at least one pressure ring (904) of the sampling module.

2. The system of claim 1, wherein the pressure ring (28; 904; 954 A-C) is configured to apply pressure for approximately 85 seconds of an approximately 15 minute sampling cycle.

3. The system of claim 1 or claim 2, wherein the sampling module (18) further includes a depth penetration control element.

4. The system of claim 3, wherein the depth penetration control element is integrated with at least one pressure ring (28; 904; 954 A-C) of the sampling module (18).

5. The system of any one of claims 1 to 4, wherein the sampling module (18) employs a lag mitigating chemical to further mitigate the ISF glucose lag.

6. The system of claim 5, wherein the lag mitigating chemical is a histamine chemical.

7. The system of any one of claims 1 to 6, wherein the sampling module (18) employs:

ultrasound; heat; vacuum; an electropotential or non-oscillatory mechanical manipulation of the body to further mitigate the ISF glucose lag.

8. The system of any one of claims 1 to 7, wherein the cartridge (12) is disposable.


**Patentansprüche**

1. System (10) zum Herausziehen einer Probe interstitiellen Fluides (ISF) und zum Überwachen eines Analyten darin, wobei das System aufweist:

eine Kassette (12), die umfaßt:

ein Probennahmemodul (18), das dazu ausgelegt ist, eine ISF-Probe aus einem Zielort (TS) eines Körpers (B) herauszuziehen,
und
einem Analysemodul (20), das dazu ausgelegt ist, einen Analyten in der ISF-Probe zu messen; und

ein lokales Controllermodul (14) in elektronischer Kommunikation mit der Kassette (12), wobei der lokale Controller so ausgestaltet ist, daß er Meßdaten von dem Analysemodul (20) empfängt und die Daten speichert,

wobei:

das Probennahmemodul (18) ein Eindring-Element und wenigstens einen Druckring (28; 904; 954A - C), der zum Aufgeben von Druck auf den Körper (B) in der Nähe des Zielortes (TS) ausgelegt ist, umfaßt, und das Probennahmemodul (18) so ausgestaltet ist, daß der wenigstens eine Druckring (28; 904; 954A - C) in der Lage ist, den Druck oszillierend aufzugeben, wodurch ein ISF-Glukosenachlauf der ISF-Probe, die von dem Probennahmemodul (18) herausgezogen worden ist, abgemildert wird,

**dadurch gekennzeichnet, daß** das Eindring-Element (902) in bezug auf den wenigstens einen Druckring (904) des Probennahmemoduls fest ist.

2. System nach Anspruch 1, bei dem der Druckring (28; 904; 954A - C) so ausgelegt ist, daß er Druck über ungefähr 85 Sekunden eines ungefähr 15 Minuten langen Probennahmezyklus ausübt.

3. System nach Anspruch 1 oder Anspruch 2, bei dem das Probennahmemodul (18) weiter ein Tiefeneindring-Steuerelement umfaßt.

4. System nach Anspruch 3, bei dem das Tiefeneindring-Steuerelement in wenigstens einen Druckring (28; 904; 954A - C) des Probennahmemoduls (18) eingebunden ist.

5. System nach einem der Ansprüche 1 bis 4, bei dem das Probennahmemodul (18) eine den Nachlauf abmildernde Chemikalie benutzt, um den ISF-Glukosenachlauf weiter abzumildern.

6. System nach Anspruch 5, bei dem die den Nachlauf abmildernde Chemikalie eine Histamin-Chemikalie ist.

7. System nach einem der Ansprüche 1 bis 6, bei dem das Probennahmemodul (18) benutzt: Ultraschall; Wärme; Vakuum; ein Elektropotential oder eine nicht oszillatorische mechanische Manipulation des Körpers, um weiter den ISF-Glukosenachlauf abzumildern.

8. System nach einem der Ansprüche 1 bis 7, bei dem die Kassette (12) ein Einwegartikel ist.

**Revendications**

1. Système d'extraction (10) d'un échantillon de fluide interstitiel (ISF, Interstitial Fluid) et surveillance d'un analyte présent dans ledit fluide, le système comprenant :

une cartouche (12) comprenant :

un module d'échantillonnage (18) adapté pour extraire un échantillon de ISF d'un site cible (TS, target site) d'un corps (B, body) et
un module d'analyse (20) adapté pour mesurer un analyte dans l'échantillon de ISF ; et
un module de contrôle local (14) en communication électronique avec la cartouche (12), le contrôleur local étant configuré pour recevoir des résultats de mesure du module d'analyse (20) et stocker les mesures,

dans lequel :

le module d'échantillonnage (18) comprend un élément de pénétration et au moins un anneau de pression (28 ; 904 ; 954A-C) adapté pour appliquer de la pression au corps (b) au voisinage du site cible (TS), et
le module d'échantillonnage (18) est configuré de sorte qu'au moins un anneau de pression (28 ; 904 ; 954A-C) soit capable d'appliquer la pression de manière oscillante pour que le temps de réponse de ISF glucose de l'échantillon de ISF extrait par le module d'échantillonnage (18) soit limité,

**caractérisé en ce que** l'élément de pénétration (902) est fixé par rapport à au moins un anneau de pression (904) du module d'échantillonnage.

2. Système selon la revendication 1, dans lequel l'anneau de pression (28 ; 904 ; 954A-C) est configuré pour appliquer de la pression pendant approximativement 85 secondes d'un cycle d'échantil-lonnage d'approximativement 15 minutes.

3. Système selon la revendication 1 ou la revendication 2, dans lequel le module d'échantil-lonnage (18) comprend en outre un élément de contrôle de pénétration en profondeur.

4. Système selon la revendication 3, dans lequel l'élément de contrôle de pénétration en profondeur est intégré avec au moins un anneau de pression (28 ; 904 ; 954A-C) du module d'échantillonnage (18).

5. Système selon l'une quelconque des revendications 1 à 4, dans lequel le module d'échantillonnage (18) emploie un produit chimique limitant le temps de réponse pour limiter encore le temps de réponse du ISF glucose.

6. Système selon la revendication 5, dans lequel le produit chimique limitant le temps de réponse est un produit chimique de type histamine.

7. Système selon l'une quelconque des revendications 1 à 6, dans lequel le module d'échantillonnage (18) emploie : les ultrasons ; la chaleur ; le vide ; un potentiel électrique ou une manipulation mécanique non oscillatoire du corps pour encore limiter le temps de réponse de ISF glucose.

8. Système selon l'une quelconque des revendications 1 à 7, dans lequel la cartouche (12) est jetable.

BS

Remote Controller
Module — 16

Local Controller
Module — 14

10

Analysis Module — 20

— 12

Sampling Module — 18

B

**FIG. 1**

ISF Flow
(To Analysis Module 20)

— 24

28 28

B

ISF ISF

18

22

TS

**FIG. 2**

FIG. 3

FIG. 4

EP 1 528 891 B1

```
                                    ┌ ─ ─ ─ ─ ─ ─ ─ ─ ─ ┐
                                    │  Local Controller │
                                    │      Module       │
                                    │        14         │
                                    └ ─ ─ ─ ─ ─ ─ ─ ─ ─ ┘
```

16

| Second Data Display 508 | Second Data Storage Element 506 | Predictive Algorithm 510 |

| Alarm 512 | Second Electronic Controller 502 | Second RF Link 504 |

| Data Carrying Element 518 | Blood Glucose Measurement System 514 | Blood Glucose Strip 516 |

BS

FIG. 5

FIG. 6

FIG. 7

FIG. 8

900

906

908

904

902

A

**FIG. 9**

950

952

954C

954B

954A

**FIG. 10**

**FIG. 11**

**FIG. 12**

1000

Providing an ISF Extraction Device
that Includes a Penetration Member
and a Pressure Ring(s)
— 1010

Contacting the Pressure Ring(s) with
a User's Skin Layer
— 1020

Penetrating the User's Skin Layer with
the Penetration Member
— 1030

Extracting ISF while Applying Pressure
to the User's Skin Layer with the
Pressure Ring(s) in a Manner that
Mitigates ISF Glucose Lag
— 1040

FIG. 13

FIG. 14

FIG. 15

Control ISF Glucose vs. Finger Blood Glucose

FIG. 16A

Test ISF Glucose vs. Finger Blood Glucose

FIG. 16B

FIG. 17

FIG. 18

Control ISF Error vs. Lag Time

FIG. 19A

Test ISF Error vs. Lag Time

FIG. 19B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1260815 A
- US 20020022789 A
- US 20020082522 A
- US 5165418 A
- WO 9707734 A
- US 20030060784 A1
- US 185605 A
- US 20050025839 A
- US 690083 A
- US 6251083 B
- US 5139023 A
- US 5231975 A
- US 5458140 A
- US 6332871 B
- US 6319210 B
- US 6240306 B
- US 6155992 A
- US 6514718 B
- US 6329161 B
- US 6702857 B
- US 6558321 B
- US 6232130 B
- US 6040194 A
- US 20040253736 A
- US 652464 A
- US 20050085839 A